(19) Europäisches Patentamt
European Patent Office
Office européen des brevets

(11) **EP 1 508 297 B1**

(12) **EUROPEAN PATENT SPECIFICATION**

(45) Date of publication and mention
of the grant of the patent:
**22.10.2008  Bulletin 2008/43**

(51) Int Cl.:
*A61B 5/024* (2006.01)    *A61B 5/22* (2006.01)

(21) Application number: **04018414.5**

(22) Date of filing: **03.08.2004**

(54) **Device for providing calorie consumption information for women**

Gerät zur Anzeige von Kaloriekonsuminformation für Frauen

Dispositif de fourniture d'information de consommation calorique pour les femmes

(84) Designated Contracting States:
**DE FR GB**

(30) Priority: **19.08.2003   JP 2003295452**

(43) Date of publication of application:
**23.02.2005   Bulletin 2005/08**

(73) Proprietor: **TANITA CORPORATION**
**Tokyo (JP)**

(72) Inventor: **Kodama, Miyuki**
**Tokyo (JP)**

(74) Representative: **Grünecker, Kinkeldey,**
**Stockmair & Schwanhäusser**
**Anwaltssozietät**
**Leopoldstrasse 4**
**80802 München (DE)**

(56) References cited:
**EP-A- 1 219 238        EP-A- 1 238 633**
**EP-A- 1 275 341**

**Description**

**Background of the Invention:**

**Field of the Invention:**

The present invention relates to a device for providing body consumption information of a woman.

**Prior Art:**

A device for providing body consumption information such as basal metabolism, energy consumption due to exercise, total energy consumption, etc. has been known in the art. Examples of such device are: a calorie consumption meter for providing calorie consumption (or energy consumption due to exercise) based on pulse rate of a person during exercise (see Patent Document 1); a burned fat mass calculation device for providing burned fat mass based on calorie consumption (or energy consumption due to exercise) (see Patent Document 2); and the like. Those devices are effective in that calorie consumption (or energy consumption due to exercise) and total burned fat mass can simply be provided.
On the other hand, as the result of search and examination conducted by the present inventor it was found that the body consumption information of a woman may be changed with physiological body transition even if the condition for causing consumption in the body (e.g. age, body weight, and other body features, as well as intensity of exercise) is kept same.
The patent documents associated with the present invention are as follows:

Patent Document 1: Japanese Patent Laid-Open No. 8-52119; and
Patent Document 2: Japanese Patent Laid-Open No. 2000-316833.

Although the previous calorie consumption meter and the burned fat mass calculation device are effective in that calorie consumption and total burned fat mass can simply be get, as described above, they have deficiency in the following point of view. That is to say, these previous devices may not necessarily provide precise or sufficient body consumption information if they are used by women because of no conditions specific for women taken into account.
In view of the above, an object of the present invention is to provide a new and improved device for providing body consumption information, which is capable of producing highly precise body consumption information by taking into account of woman's physiological body transition terms.

**Summary of the Invention:**

In order to attain such object the present invention provides a device for providing body consumption information of a woman, comprising: a basic body data acquisition unit; a number-of-days-after-menstruation acquisition unit; a body transition term data storage unit; a body consumption information calculation formula storage unit; a body transition term selection unit; a body consumption information calculation unit; and a body consumption information display unit, wherein said basic body data acquisition unit acquires basic body data relating to consumption in the body,
said number-of-days-after-menstruation acquisition unit acquires the number of days that have elapsed since a menstruation start day,
said body transition term data storage unit stores, in advance, the body transition term data that is formed by correlation of woman's physiological body transition terms with the number of days after menstruation,
said body consumption information calculation formula storage unit stores, in advance, an calculation formula for getting information about consumption in the body, taking into account of woman's physiological body transition term, for each of the body transition terms,
said body transition term selection unit selects a woman's physiological body transition term corresponding to the number of days after menstruation acquired by said number-of-days-after-menstruation acquisition unit on the basis of the body transition term data stored in said body transition term data storage unit,
said body consumption information calculation unit calculates information about consumption in the body, taking into account of woman's physiological body transition terms, by substituting the basic body data relating to body information acquired by said basic body data acquisition unit for the calculation formula retrieved from said body consumption information calculation formula storage unit according to the woman's physiological body transition term selected by said body transition term selection unit, and
said body consumption information display unit displays the body information, taking into account of woman's physiological body transition term, calculated by said body consumption information calculation unit.
According to an embodiment of the present invention the device for providing body consumption information of a woman further comprises: a target basic data acquisition unit; a target information calculation formula storage unit; a target information calculation unit; and a target information display unit, wherein

said target basic data acquisition unit acquires a target basic data relating to consumption in the body,

said target information calculation formula storage unit stores, in advance, an calculation formula for providing target information, taking into account of woman's physiological body transition term, for each of body transition terms,

said target information calculation unit calculates target information, taking into account of woman's physiological body transition term, by substituting the target basic data relating to consumption in the body acquired by said target basic data acquisition unit for the calculation formula retrieved from said target information calculation formula storage unit according to the woman's physiological body transition term selected by said body transition term selection unit, and

said target information display unit displays the target information, taking into account of woman's physiological body transition term, calculated by said target information calculation unit.

According to another embodiment of the present invention the device for providing body consumption information of a woman further comprises an advice information display unit, said advice information display unit displays advice information for consumption in the body, together with the body consumption information calculated by said body consumption information calculation unit in comparison with the target information calculated by said target information calculation unit.

According to further embodiment of the present invention the device for providing body consumption information of a woman further comprises an intensity-of-exercise measurement unit for measuring how much an exercise has been done, said body consumption information calculation unit calculates the body consumption information, taking into account of the intensity of exercise measured by said intensity-of-exercise measurement unit.

According to yet further embodiment of the present invention the device for providing body consumption information of a woman further comprises a time-of-exercise measurement unit for measuring how long an exercise has been done, said body consumption information calculation unit calculates the body consumption information, taking into account of the time duration of exercise measured by said time-of-exercise measurement unit.

According to yet further embodiment of the present invention said intensity of exercise is represented by the number of steps.

According to yet further embodiment of the present invention said time duration of exercise is represented by the walking time.

According to yet further embodiment of the present invention the device for providing body consumption information of a woman further comprises: a pulse rate measurement unit; and a pitch guide tone generator unit, wherein said pulse rate measurement unit measures a pulse rate of a woman under test, and

said pitch guide tone generator unit generates pitch guide tone for prompting the woman under test to walk at pulse rate of fixed and safety range according to the pulse rate measured by said pulse rate measurement unit.

According to yet further embodiment of the present invention said number-of-days-after-menstruation acquisition unit comprises an input section and a clock section, said input unit enters the previous menstruation date and the current date, and said clock section calculates the number of days after menstruation, which means the number of days elapsed from the previous menstruation date to the current date, depending on the previous menstruation date and the current date entered by said input unit.

According to yet further embodiment of the present invention said body transition term includes follicular phase and luteal phase.

According to yet further embodiment of the present invention said body transition term includes menstruation term, diet term, before-and-after ovulation term, PMS prevention term, and PMS term.

According to yet further embodiment of the present invention said body consumption information includes at least one of the followings: basal metabolism, energy consumption due to exercise, total energy consumption, fat mass burned due to exercise, and total burned fat mass.

According to yet further embodiment of the present invention said target information includes target number of steps.

The device for providing body consumption information of a woman according to the present invention provides body consumption information by taking into account of woman's physiological body transition terms, and therefore, it can provide highly precise body consumption information for a woman, which is effective in control of body consumption or in health care.

In addition, the device for providing body consumption information of a woman according to the present invention provides target information by taking into account of woman's physiological body transition terms, and it provides advice information with respect to the target information, which is effective in continuation of more proper health care.

**Brief Description of the Drawings:**

Now, the present invention will be described in more detail with reference to the accompanying drawings, in which:

Fig. 1 is a schematic external view of a device for providing body consumption information of a woman according to an embodiment of the present invention;

Fig. 2 is a schematic view of the device in Fig. 1, illustrating one example of actual usage where the device is worn on the body of a woman;

Fig. 3 is a block diagram illustrating internal and external components of the device in Fig. 1;

Fig. 4 is a main flow chart for explaining entire operation of the device in Fig. 1;

Fig. 5 is a subroutine flow chart for explaining body information and other basic data setting and registration mode of the device in Fig. 1;

Fig. 6 is a subroutine flow chart for explaining target information calculation mode of the device in Fig. 1;

Fig. 7 is a subroutine flow chart for explaining body consumption information calculation mode of the device in Fig. 1;

Fig. 8 is a view illustrating one example of a display screen for entering basic body data;

Fig. 9 is a view illustrating one example of a display screen for entering basic menstruation data;

Fig. 10 is a view illustrating one example of a display screen for entering target basic data;

Fig. 11 is a view illustrating one example of a display screen for measuring pulse rate at rest condition;

Fig. 12 is a view illustrating one example of a display screen for representing information about menstruation related body transition terms before measurements of number of steps, etc.;

Fig. 13 is a view illustrating one example of a display screen for representing target information and health related information before measurements of number of steps, etc.;

Fig. 14 is a view illustrating one example of a display screen for representing number of steps, energy consumption due to exercise and fat mass burned due to exercise;

Fig 15 is a view illustrating one example of a display screen for representing target number of steps, actual number of steps and advice information;

Fig. 16 is a view illustrating one example of a display screen for graphically representing historical data of consumption in body;

Fig. 17 is a view illustrating one example of a display screen for representing historical data of menstruation data and number of days for menstruation period in the form of a table;

Fig. 18 is a view illustrating one example of a display screen for representing data of anticipated term for start of future menstruation;

Fig. 19 is a view illustrating one example of a display screen for representing body consumption information taking into account of woman's physiological body transition terms;

Fig. 20 shows a table 1 representing age · sex coefficients used in the calculation formulae;

Fig. 21 shows a table 2 representing relation between number of days from menstruation and body transition terms; and

Fig. 22 shows a table 3 representing relation between time period for one step and calorie constant.

## Description of the Preferred Embodiments:

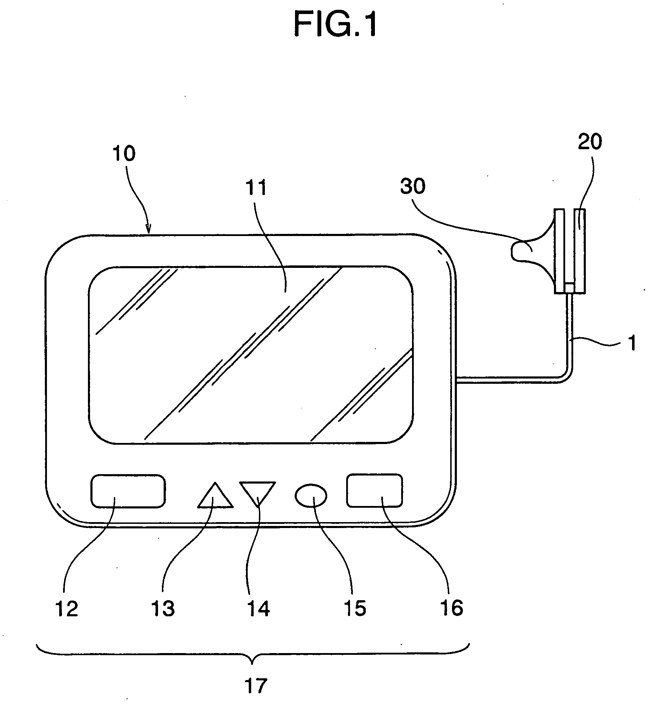

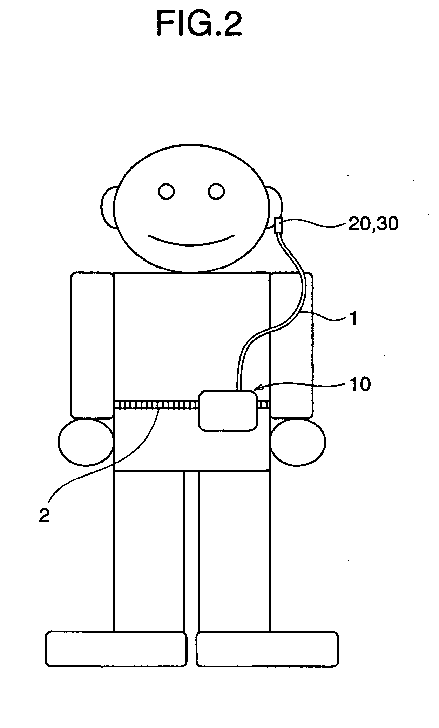

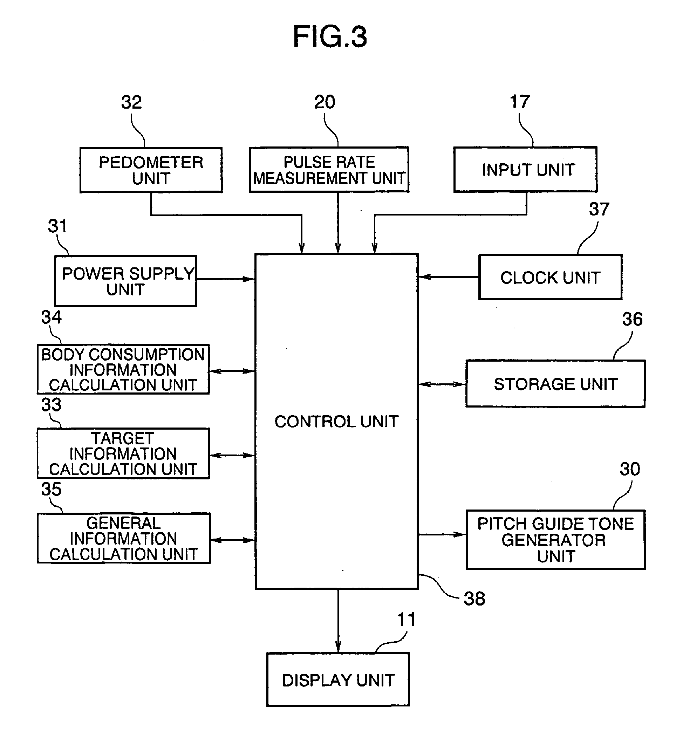

Fig. 1 is a schematic external view of a device for providing body consumption information of a woman according to an embodiment of the present invention; Fig. 2 is a schematic view of the device for providing body consumption information of a woman in Fig. 1, illustrating one example where the device is worn on the body of a woman; and Fig. 3 is a block diagram illustrating internal components of the device for providing body consumption information of a woman in Fig. 1. Referring to Fig. 1, the device for providing body consumption information of a woman according to this embodiment comprises: a case body 10; a pulse sensor 20 forming a part of a pulse rate measurement unit coupled to the case body 10 via a cord 1 having suitable length; and a earphone 30 forming a pitch guide tone generator unit. Provided on an external surface of the case body 10 are: a display unit 11; and an input unit 17 including a start button 12, an up-button 13, a down-button 14, a setting button 15, and a switching button 16. Although not shown, on the rear side of the case body 10, there is provided a clip for mounting the case body 10 to a belt 2 of a user, for example, as seen in Fig. 2. In addition, a battery lid is provided for replacing batteries forming power supply unit for the device. As shown in Fig. 2, the pulse sensor 20 and the earphone 30 are configured to wear on a lobe of the user, for example, when the device is in use.

As is apparent in Fig. 3, the case body 10 contains: a pulse rate measurement unit 20 including the pulse sensor, an A/D converter and a pulse rate calculation unit; a pitch guide tone generator unit 30 including an earphone; a power supply unit 31 including batteries; a pedometer unit 32 including an accelerator sensor, an A/D converter and a number-of -steps calculation unit; a target information calculation unit 33; a body consumption information calculation unit 34; a general information calculation unit 35; a storage unit 36; a clock unit 37; and a control unit 38.

Now, detailed description of configuration and function for each of the units will be made. At first, the power supply unit 31 is made up of batteries for providing electric power to each of electrical units.

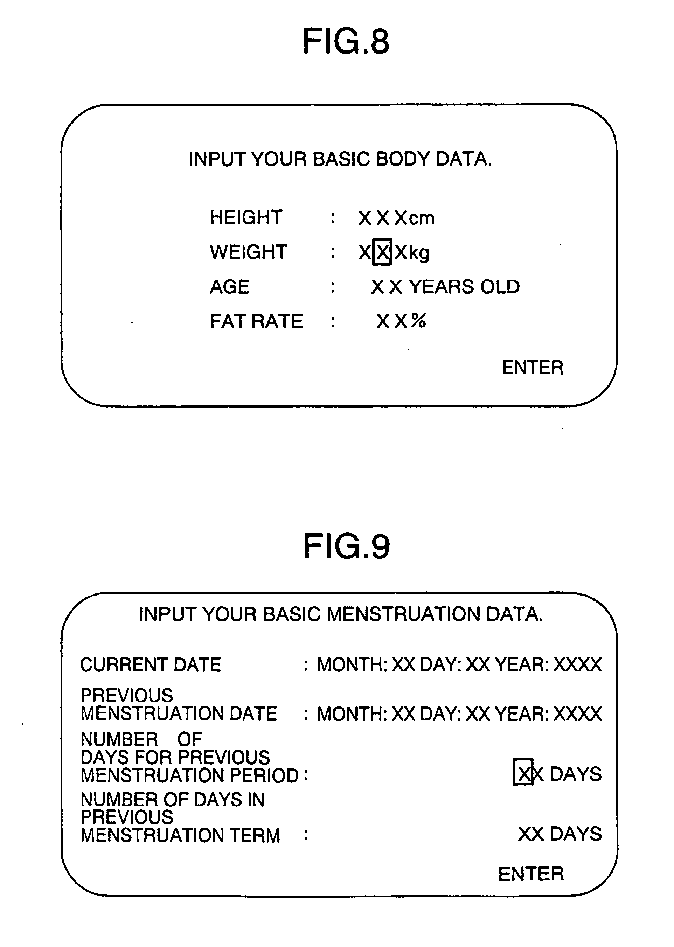

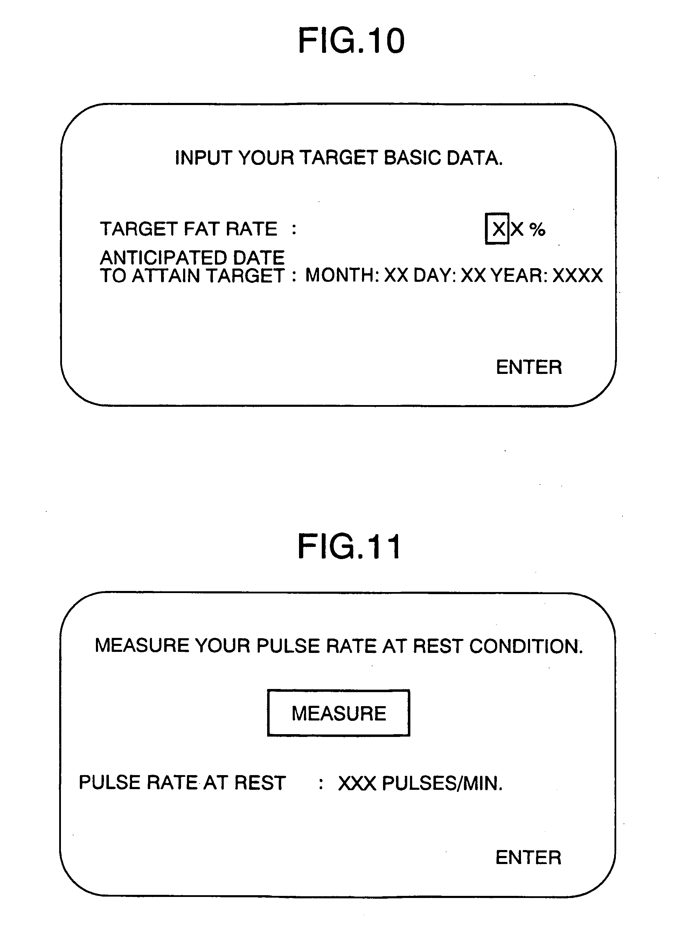

The input unit 17 is configured to operate the start button 12, the up-button 13, the down-button 14, the setting button 15, and the switching button 16 in order to enter the following data: basic body data of a woman under test (height, body weight, age and fat rate all at current time), as shown in Fig. 8; basic menstruation data of the woman (current date, previous menstruation date, number of days for the previous menstruation period, and number of days within the previous menstruation term), as shown in Fig. 9; and target basic data (target fat rate and anticipated date on which the target is attained), as shown in Fig. 10. It is noted that a square mark on a display screen of the display unit 11, as shown in Fig. 8, for example, means a cursor.

The pedometer unit 32 includes a detector, an A/D converter and a number-of-steps calculator for determining the number of steps during exercise (at the time of activity) in the same manner as the previous pedometer.

The pulse rate measurement unit 20 incorporating a pulse sensor is made up of a detector, an A/D converter and a pulse rate calculator to determine pulse rate of the woman at rest condition in the same manner as the previous pulse rate meter.

The clock unit 37 includes a clock and a calculator for determining time data such as walking time, number of days within target interval, number of days after menstruation, etc.

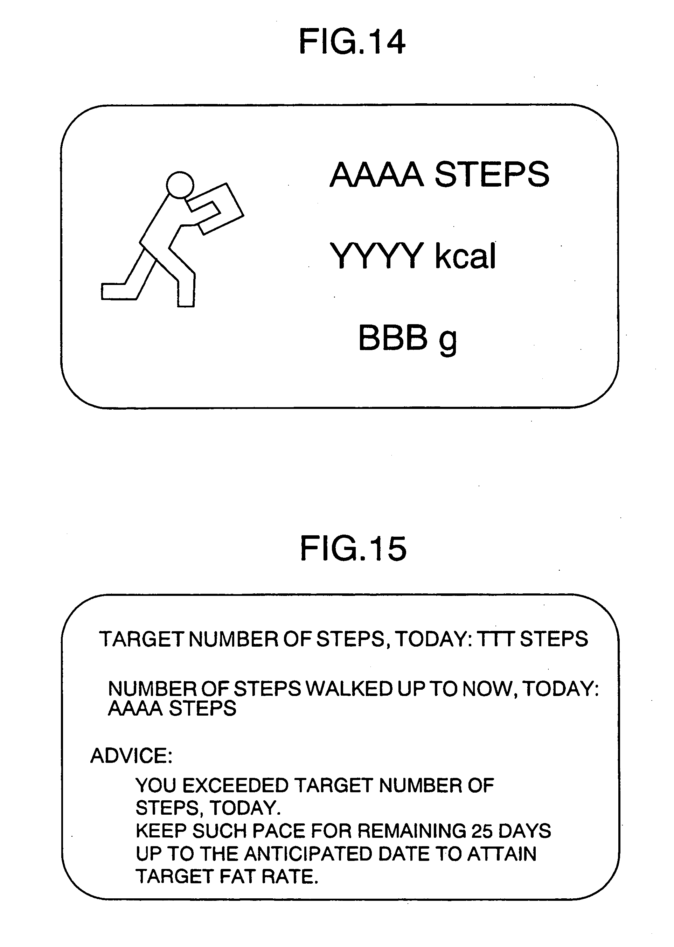

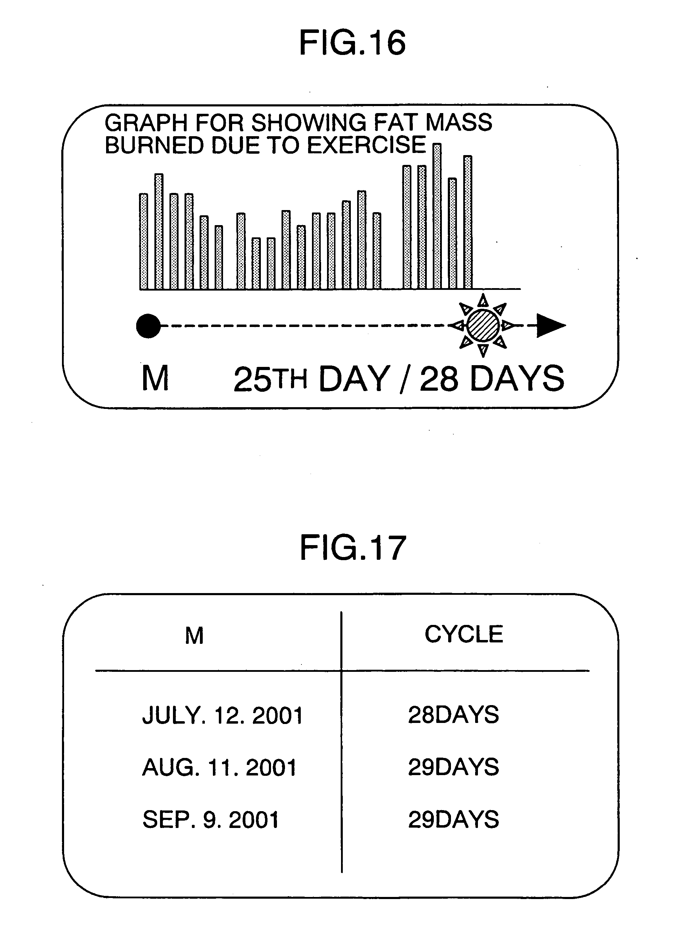

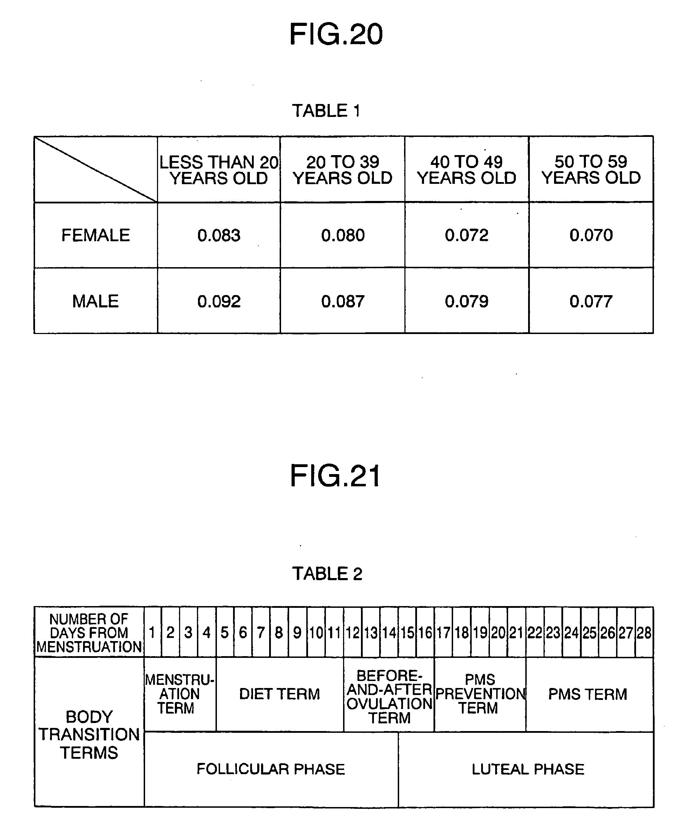

The storage unit 36 stores the following calculation formulae: formulae (1) to (9) for calculating target information (target number of steps per a day); and formulae (11) to (37) and (41) to (57) for calculating body consumption information (basal metabolism, energy consumption due to exercise, total energy consumption, fat mass burned due to exercise, and total burned fat mass), taking into account of woman's physiological body transition term (follicular phase and luteal phase). Coefficients used in those formulae are also stored in the storage unit 36. Furthermore, the storage unit 36 stores the following information in advance or if necessary: relation (or body transition term data) between number of days after menstruation and body transition term, as listed in table 2 in Fig. 21; advice information according to the condition when actual number of steps reaches the target number of steps, as shown in Fig. 15; historical graphic information about body consumption, as shown in Fig. 16; historical information about menstruation date and number of days for menstruation period, as shown in Fig. 17; each type of input information, as shown in Figs. 8 to 10; historical information about pulse rate at rest condition measured by the pulse rate measurement unit, as shown in Fig. 11; and historical information about number of steps measured by the pedometer unit 32.

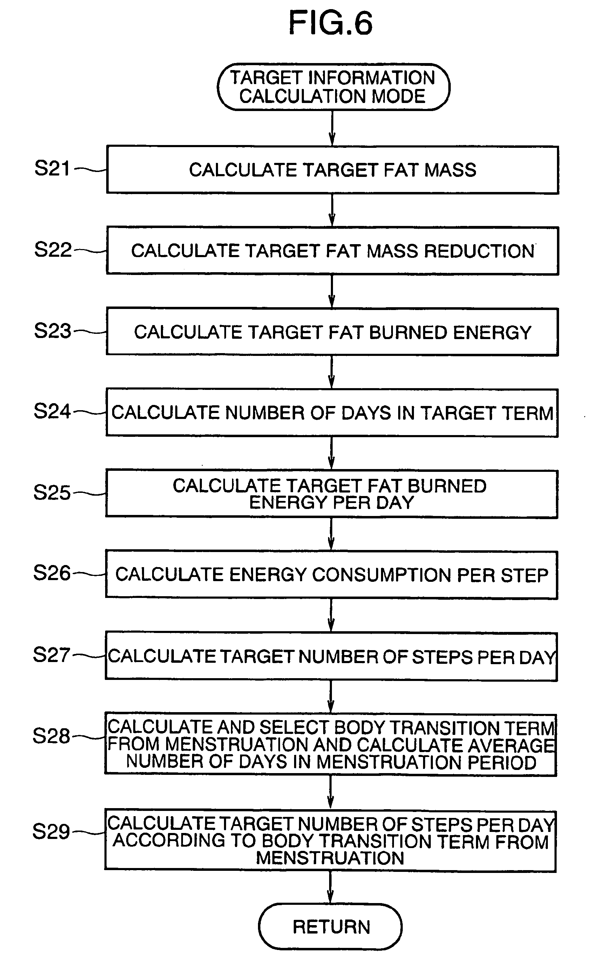

The target information calculation unit 33 calculates target number of steps per a day depending on the body transition terms from menstruation by substituting each type of data from the input unit 17, the pedometer unit 32 and the clock unit 37 for formulae (1) to (7) of the following formulae (1) to (9) stored in the storage unit 36 according to the flow in target information calculation mode in Fig. 6.

$$\text{Target Fat Mass [kg]} = \frac{\dfrac{TargetFatRate[\%]}{100} \times (CurrentWeight[kg] - \dfrac{CurrentFatRate[\%]}{100} \times CurrentWeight[kg])}{1 - \dfrac{TargetFatRate[\%]}{100}} \quad (1)$$

$$\text{Target Fat Reduction [kg]} = \text{Current Weight [kg]} \times \text{Current Fat Rate [\%]} / 100 - \text{Target Fat Mass [kg]} \quad (2)$$

$$\text{Target Fat Burned Energy [kcal]} = \text{Target Fat Reduction [kg]} \times 7200 \text{[kcal/kg]} \quad (3)$$

$$\text{Target Fat Burned Energy per Day [kcal/day]} = \text{Target Fat Burned Energy [kcal]} / \text{Number of Days in Target Interval [days]} \quad (4)$$

Energy Consumption due to Exercise per Step [kcal/step] =

Age · Sex Coefficient × Weight [kg] ∕ 100        (5)

Target Number of Steps per Day [steps/day] =

Target Fat Burned Energy per Day [kcal/day] ∕ Energy

Consumption due to Exercise per Step [kcal/step]       (6)

Target Number of Steps per Day in Luteal Phase [steps/day] =

Target Number of Steps per Day [steps/day] ×

Luteal Phase Correction Coefficient        (7)

Target Number of Steps per Day in Follicular Phase

(in Menstruation Term) [steps/day] = Target Number

of Steps per Day [steps/day] × Follicular Phase

(in Menstruation Term) Correction Coefficient       (8)

Target Number of Steps per Day in Follicular Phase

(after Menstruation Term) [steps/day] = Target Number

of Steps per Day [steps/day] + Target Number of Steps per Day

[steps/day] × Follicular Phase (after Menstruation Term) Correction

Coefficient × 14 ∕ (Average Number of Days for Menstruation Period

[days] − 14 + Number of Days within Menstruation Term [days] )    (9)

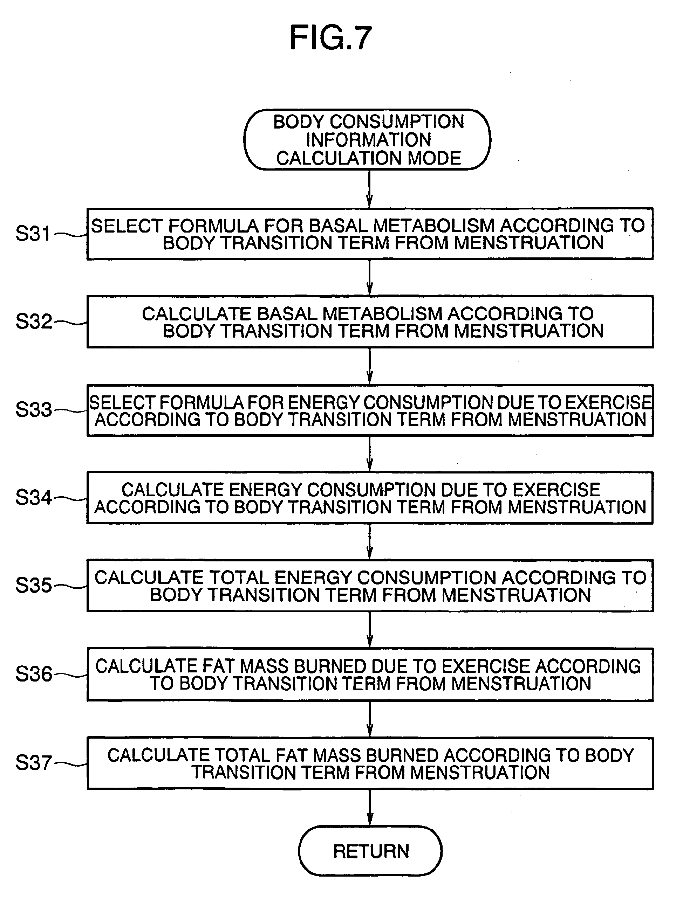

The body consumption information calculation unit 34 calculates body consumption information (basal metabolism, energy consumption due to exercise, total energy consumption, fat mass burned due to exercise, and total burned fat mass), taking into account of woman's physiological body transition term (follicular phase and luteal phase) by substituting each type of data from the input unit 17, the pedometer unit 32, the pulse rate measurement unit 20 and the clock unit 37 for the following formulae (11) to (23) stored in the storage unit 36 according to the flow in body consumption information calculation mode in Fig. 7.

Fat Free Mass [kg] = Weight [kg] − Weight [kg] × Fat Rate [%] ∕ 100    (11)

Basal Metabolism [kcal/day] = Coefficient "a" × Fat Free Mass$^2$ [kg$^2$] +

Coefficient "b" × Fat Free Mass [kg] + Coefficient "c" / Age[years] +

Coefficient "d" × Weight [kg] + Coefficient "e"  (12)

Basal Metabolism in Follicular Phase [kcal/day] = Coefficient "f1" ×

Basal Metabolism [kcal/day] + Coefficient "g1"  (13)

Basal Metabolism in Luteal Phase [kcal/day] = Coefficient "f2" ×

Basal Metabolism [kcal/day] + Coefficient "g2"  (14)

Energy Consumption due to Exercise [kcal/day] = Age · Sex Coefficient ×

Weight [kg] × Number of Steps [steps] / 100  (15)

Energy Consumption due to Exercise in Follicular Phase [kcal/day] =

Coefficient "h1" × Energy Consumption due to Exercise [kcal/day]

+ Coefficient "i1"  (16)

Energy Consumption due to Exercise in Luteal Phase [kcal/day] =

Coefficient "h2" × Energy Consumption due to Exercise [kcal/day]

+ Coefficient "i2"  (17)

Total Energy Consumption in Follicular Phase [kcal/day] =

Basal Metabolism in Follicular Phase [kcal/day] + Energy

Consumption due to Exercise in Follicular Phase [kcal/day]  (18)

Total Energy Consumption in Luteal Phase [kcal/day] =

Basal Metabolism in Luteal Phase [kcal/day] + Energy

Consumption due to Exercise in Luteal Phase [kcal/day]  (19)

Intensity of Exercise [%] = Coefficient "j" × Number of Steps (steps) /

Walking Time [min] + Coefficient "k"  (20)

$$\text{Fat Burning Efficiency [\%]} = -\text{Coefficient "m"} \times \text{Intensity of Exercise [\%]}$$
$$-\text{Coefficient "n"} \times \text{Pulse Rate at Rest Condition [pulses/min]} +$$
$$\text{Coefficient "p"} \times \text{Walking Time [min]} - \text{Coefficient "q"} \times$$
$$\text{Sex Coefficient} + \text{Coefficient "r"} \tag{21}$$

$$\text{Fat Mass Burned Due to Exercise in Follicular or Luteal Phase [g]} =$$
$$\text{Fat Burning Efficiency [\%]} \times \text{Energy Consumption due to Exercise}$$
$$\text{in Follicular or Luteal Phase [kcal/day]} / 7.2 \text{ [kcal/g]} \tag{22}$$

$$\text{Total Fat Mass Burned in Follicular or Luteal Phase [g]} = \text{Fat Mass}$$
$$\text{Burned Due to Exercise [g]} +$$
$$\text{Basal Metabolism in Follicular or Luteal Phase [kcal/day]} /$$

$$7.2 \text{ [kcal/g]} \tag{23}$$

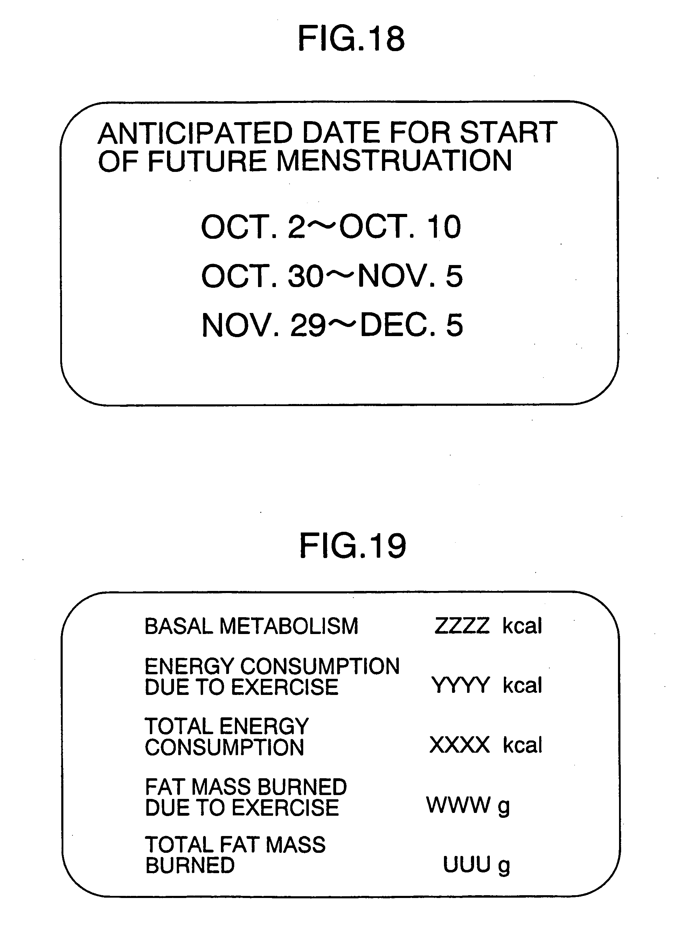

The general information calculation unit 35 calculates some general information (e.g. interval between pitch guide tones generated by the pitch guide tone generator unit 30 for guiding a user to walk at pulse rate of fixed and safety range, anticipated term for start day of future menstruation, as shown in Fig. 18, etc.) other than those information calculated in the target information calculation unit 33 and the body consumption information calculation unit 34.

The pitch guide tone generator unit 30 generates pitch guide tones for guiding a user to walk at pulse rate of fixed and safety range under the control of the control unit 38, as described later.

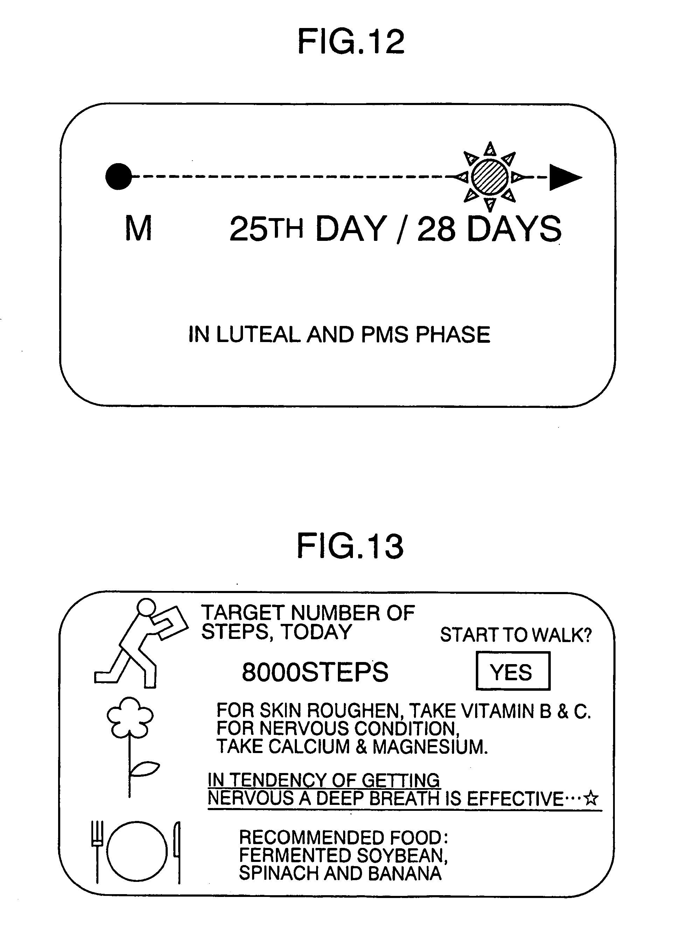

The display unit 11 displays the following data and information: basic body data, basic menstruation data and target basic data entered via the input unit 17, as shown in Figs. 8 to 10; pulse rate at rest condition measured by the pulse rate measurement unit 20, as shown in Fig. 11; body transition term data associated with menstruation, which is displayed before measurement of number of steps, etc., as shown in Fig. 12; target information (target number of steps on current day) and health related information, which is displayed before measurement of number of steps, etc., as shown in Fig. 13; number of steps, energy consumption due to exercise, and fat mass burned due to exercise measured by the pedometer 32, as shown in Fig. 14; target number of steps on current day, number of steps walked up to now on current day (actual number of steps), and advice information, which are displayed by operation of the switching button 16 after measurement with the pedometer unit 32, as shown in Fig. 15; historical information of body consumption (fat mass burned due to exercise) in the form of graph, which is displayed by operation of the switching button 16 after measurement with the pedometer unit 32, as shown in Fig. 16; historical information of menstruation date and number of days for menstruation period, which is displayed by operation of the switching button 16 after measurement with the pedometer unit 32, as shown in Fig. 17; information of anticipated term for start day of future menstruation (next time, second and third subsequent times), which is displayed by operation of the switching button 16 after measurement with the pedometer unit 32, as shown in Fig. 18; and body consumption information (basal metabolism, energy consumption due to exercise, total energy consumption, fat mass burned due to exercise, and total burned fat mass), taking into account of woman's physiological body transition terms (follicular phase and luteal phase), which is displayed by operation of the switching button 16 after measurement with the pedometer unit 32, as shown in Fig. 19.

The control unit 38 acts to control operations of the various components in the device to provide the following information: target number of steps per a day depending on body transition terms from menstruation of a woman, which is calculated in the target information calculation unit 33 according to each data sent from the input unit 17, the pedometer unit 32, the pulse rate measurement unit 20 and the clock unit 37, and each type of formula stored in the storage unit 36; body consumption information (basal metabolism, energy consumption due to exercise, total energy consumption, fat mass burned due to exercise, and total burned fat mass), taking into account of woman's physiological body transition terms (follicular phase and luteal phase), which is calculated in the body consumption information calculation unit 34;

and some general information other than those calculated in the target information calculation unit 33 and the body consumption information calculation unit 34, which general information is calculated in the general information calculation unit 35. Furthermore, the control unit 38 acts to control operation of the pitch guide tone generator unit 30 to generate the guide tone; to control the display unit 11 to provide each type of display thereon; and to control the storage unit 36 to store each type of data therein.

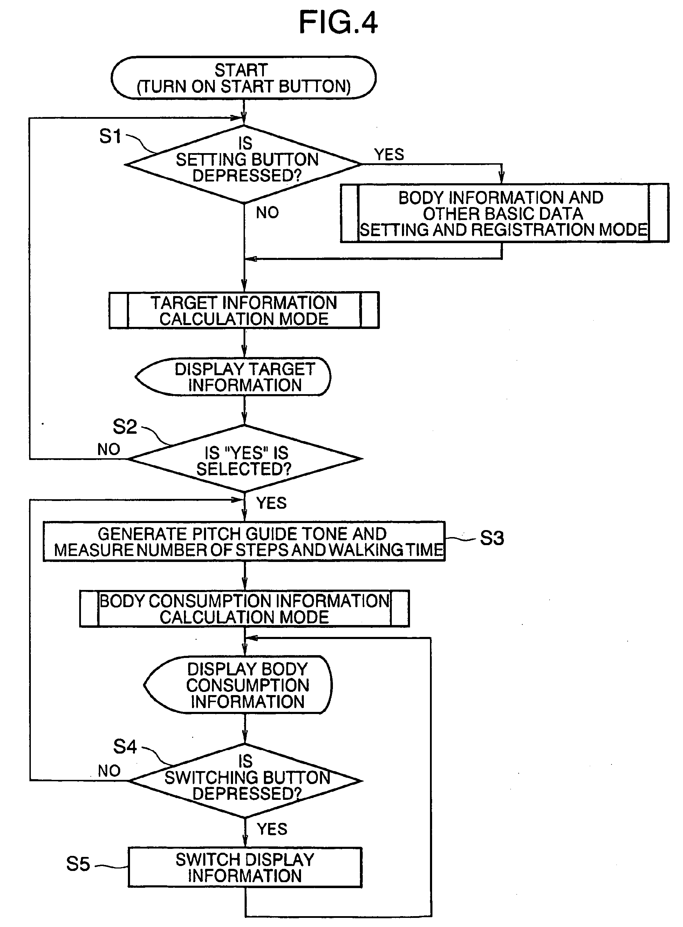

Now, entire operation of the device for providing body consumption information of a woman configured in the manner as described above will be described in more detail with reference to main flow chart of Fig. 4 and subroutine flow charts of Figs. 6 and 7. Referring first to the main flow chart of Fig. 4, after preparation in which a battery lid on the rear side of the case body 10 is opened and batteries are installed, a user depresses the start button 12. Then, electric power is supplied from the power supply 31 to each of electrical components in the device, and thereafter, at step S1, a check is made to determine whether the setting button 15 is depressed within the time period of several seconds.

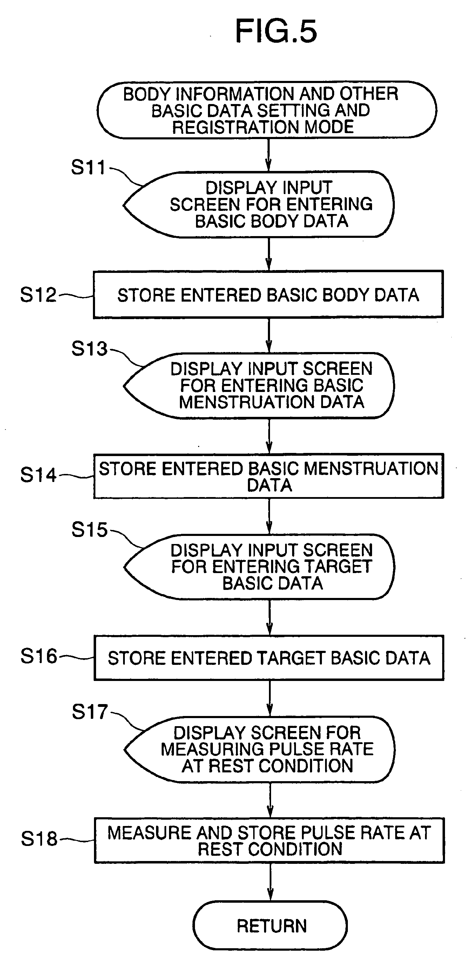

If the setting button 15 is depressed the routine enters basic data setting and registration mode in which the basic data such as body information, etc. is set and registered, which is described later with reference to Fig. 5. On the other hand, if the setting button 15 is not depressed the routine enters target information calculation mode, which is described later with reference to Fig. 6. After the target information calculation mode, the display unit 11 displays information as shown in Fig. 12 for a fixed time period, and then, the information on the screen is switched to one as shown in Fig. 13.

Referring to Fig. 12 illustrating information displayed on the screen of the display unit, "M" means previous menstruation day; "25th day" means number of days elapsed from the previous menstruation day; and "28 days" means number of days for previous menstruation period. A "solar" mark on the top of the screen is turned ON at such position that corresponds to number of days from menstruation. A message "Term is in PMS and luteal phase" indicates such body transition term that corresponds to number of days from menstruation. Then, referring to Fig. 13 illustrating information displayed on the screen of the display unit, "8000 steps" means target number of steps per a day determined depending on body transition term from menstruation; a square mark encircling "Yes" means a cursor; and a message "For skin roughen take Vitamin B, C,.........Spinach, Banana" is information about health care. "Start to walk?" on the screen in Fig. 13 is a message for asking a user whether she will start walking or not, which is associated with "Yes" or "No" indicated beneath that message. Every time the up-button 13 or the down-button 14 is depressed during the time when the information in Fig. 13 displayed on the screen then "Yes" or "No" is alternately displayed.

If, at step S2, the user depresses the setting button 15 when "Yes" is displayed on the screen in Fig. 13 then the routine proceeds to YES branch. On the other hand, if the user depresses the setting button 15 when "No" is displayed then the routine proceeds to NO branch. That is to say, if the setting button 15 is depressed when "No" is selected in Fig. 13 then the routine returns to decision block for the setting button (step S 1) after start of the operation. However, if the setting button 15 is depressed when "Yes" is selected then the routine proceeds to step S3 where generation of pitch guide tone/measurement of number of steps and walking time are performed.

At step S3 the pitch guide tone is generated by the pitch guide tone generator unit 30 in order to guide the user to walk at the pulse rate of fixed and safety range. The user or the woman under test wears the case body 10 on her body with the aid of a belt and the like and also wears the earphone on her ear. Thereafter, she starts to walk while being guided by the pitch guide tone generated by the pitch tone generator unit 30. The pedometer unit 32 starts to measure number of steps and the clock unit 37 starts to measure walking time (or time period elapsed after selection of "Yes" is determined).

Then, the routine enters body consumption information calculation mode, which is described later with reference to Fig. 7, where the body consumption information calculation unit 34 calculates body consumption information (basal metabolism, energy consumption due to exercise, total energy consumption, fat mass burned due to exercise, and total burned fat mass), taking into account of woman's physiological body transition terms (follicular phase and luteal phase).

Then, the display unit 11 displays body consumption and other information on the screen thereof, as shown in Fig. 14. The result of calculation (number of steps, calorie consumption due to exercise and fat mass burned due to exercise) provided every time when sampling of number of steps and walking time is performed is displayed on the display screen in Fig. 14. On this display screen "AAAA steps" on top row means number of steps that the user walked up to now on the current day; "YYYY kcal" on middle row means calorie consumption due to exercise that the user consumed up to now on the current day; and "BBB g" on bottom row means fat mass burned due to exercise that the user burned up to now on the current day. Each of values on this display screen is updated every time the sampling is performed at step S3 in order to measure number of steps and walking time. Every time the switching button 16 is depressed, at step S4, the content of information displayed on the screen is successively changed, at step S5, in the following sequence: Fig. 14 → Fig. 15 → Fig. 16 → Fig. 17 → Fig. 18 → Fig. 19 → Fig. 14.

If the switching button 16 is not depressed, at step S4, while body consumption and other information is displayed then the routine returns to step S3 where pitch guide tone is generated and sampling is performed to measure number of steps and walking time. However, if the switching button 16 is depressed, the content of information displayed on the display unit 11 is changed from that shown in Fig. 14 to that shown in Fig. 15. Thereafter, the content of information displayed on the screen is successively changed in the sequence, as described above, every time when the switching button 16 is depressed. The values for body consumption information (basal metabolism, energy consumption due to exercise, total energy consumption, fat mass burned due to exercise, and total burned fat mass) on the display screen, as shown in Fig. 19, are updated every time the sampling is performed to measure number of steps and walking time at step S3.

Referring to Fig. 15 illustrating information displayed on the screen of the display unit, "TTTT steps" means target number of steps per a day depending on body transition term from menstruation; "AAAA steps" means number of steps that the user walked up to now on current day; and "Target number of steps is exceeded, today. .....Keep this pace for remaining 25 days........" is advice message. Referring to Fig. 16, a ''bar graph" represents historical data by plotting time period on horizontal axis and fat mass burned due to exercise on vertical axis; and "M", "25th day", "28 days" and "Solar mark" have same meaning as those described with reference to Fig. 12. Referring to Fig. 17, "M column" lists previous menstruation date; and "CYCLE column" lists previous number of days for menstruation period. Referring to Fig. 18, "10.2 ~10.10" means anticipated term during which the next menstruation will be started; "10.30~11.5" means anticipated term during which second subsequent menstruation will be started; and "11.29~12.5" means anticipated term during which third subsequent menstruation will be started. Referring to Fig. 19, "ZZZZ kcal" on first row means basal metabolism on current day; "YYYY kcal" on second row means energy consumption due to exercise that the user consumed up to now on current day; "XXXX kcal" on third row means total calorie consumption that the user consumed up to now on current day; "WWW g" on fourth row means fat mass burned due to exercise that the user burned up to now on current day; and "UUU g" on fifth row means total fat mass burned due to exercise that the user burned up to now on current day.

Referring now to subroutine flow chart of Fig. 5, data setting and registration mode of operation in which basic data such as body information and the like is set and registered will be described in more detail. When entering the data setting and registration mode, at step S11, the display unit 11 provides data input screen for entering basic body data, as shown in Fig. 8. The user enters the relevant values for data items according to the guide (or movement) of the cursor. When the cursor is positioned at "Enter" on the screen the user may depress the setting button 15 to register the values for data items on the screen in the storage unit 36, at step S 12. More particularly, the user enters the values for height, body weight, age, and fat rate, as guided by movement of the cursor, and stores (or registers) them in the storage unit. In this respect, every time the up-button 13 or the down-button 14 is depressed, the value pointed by the cursor is increased /decreased. When the setting button 15 is depressed the value pointed by the cursor is entered, and then, operation proceeds to a step of entering the next value. Finally, when the cursor is positioned at "Enter" the setting button 15 is depressed to register the values for data items on the screen in the storage unit 36. It is noted that the same process of setting and registration of the values, as described above, is again performed on the display screen as shown in Figs. 9 and 10, which is described later.

Then, at step S13, the display unit 11 provides display on the screen thereof for entering basic menstruation data, as shown in Fig. 9. At step S14, the user enters the values for current date, previous menstruation date, number of days for previous menstruation period, and number of days in previous menstruation term, as guided by the cursor, on the display screen in Fig. 9, and then, stores (or registers) them in the storage unit. Thereafter, at step S15, the display unit 11 provides display on the screen thereof for entering target basic data, as shown in Fig. 10. At step S16, the user enters the values for target fat rate and anticipated date to reach the target, as guided by the cursor, on the display screen in Fig. 10, and then, stores (or registers) them in the storage unit.

Then, at step S17, the display unit 11 provides display on the screen thereof for measuring pulse rate of the user at rest condition, as shown in Fig. 11. When measuring pulse rate at rest condition the user wears the pulse sensor on her lobe, as described with reference to Fig. 2. Every time the up-button 13 or the down-button 14 is depressed the wording pointed by the cursor is switched between "Measure" and "Not Measure". If the setting button 15 is depressed while "Measure" is selected then measurement of pulse rate at rest condition is started so that the pulse rate at rest condition is displayed at "XXX" on the screen every time the sampling is made. After a preset fixed time period is elapsed the measurement is automatically terminated and the cursor is moved to the position of "Enter" on the screen. If the setting button 15 is depressed, at step S18, then the measured value of pulse rate at rest condition is stored in the storage unit 36 and the basic data setting and registration mode is terminated. However, if the setting button 15 is depressed while ''Not Measure" is selected then the cursor is moved to the position of "Enter" without any measurement of pulse rate at rest condition conducted.

Referring now to subroutine flow chart of Fig. 6, target information calculation mode of operation will be described. When entering this mode, at step S21, the target information calculation unit 33 calculates target fat mass by substituting target fat rate, current body weight and current body fat rate entered via the input unit 17 in body information and other basic data setting and registration mode for the formula (1) retrieved from the storage unit 36.

Then, as the routine proceeds through steps S22 to S27, the target information calculation unit 33 calculates target number of steps per a day by substituting data entered via the input unit 17 (current body weight and current body fat rate), data measured by the clock unit 37 (number of days in target term that is calculated on the basis of current date and anticipated date to reach the target entered via the input unit 17 at the previous step using the formula (4)), some coefficient already stored in the storage unit 36 (one of the coefficients in "female" row in table 1 as shown in Fig. 20 is selected depending on age entered via the input unit 17), and previously calculated data for formulae (2) to (6) retrieved from the storage unit 36. More precisely, at step S22, the formula (2) is used to calculate target reduction of fat mass, and at step S23, the formula (3) is used to calculate target fat burned energy. Then, at step S24, number of days from current date to anticipated date to reach the target is counted to provide number of days in the target term. Then, at step S25, the formula (4) is used to calculate target fat burned energy per a day, and at step S26, the formula (5) is used to calculate energy consumption per a step. Then, at step S27, the formula (6) is used to calculate the target number of steps per a day.

Then, at step S28, the clock unit 37 counts days between the previous menstruation date and the current date both entered via the input unit 17 to determine number of days from menstruation, which is then used to select body transition term corresponding thereto in table 2 in Fig. 21. The target information calculation unit 33 operates to average the historical data of number of days for menstruation period stored in the storage unit 36 to provide an average number of days for menstruation period.

Then, at step S29, the calculated data (target number of steps per a day and average number of days for menstruation period), coefficients stored in the storage unit (follicular phase (menstruation term) correction coefficient, luteal phase correction coefficient, and follicular phase (after menstruation term) correction coefficient), and number of days in menstruation term (number of days in previous menstruation term) entered via the input unit are substituted for any one of formulae (7) to (9) adaptable to the selected body transition term and the average number of days for menstruation period to calculate target number of steps per a day according to the body transition term from menstruation (luteal phase, follicular phase (menstruation term) and follicular phase (after menstruation term)).

Referring now to subroutine flow chart of Fig. 7, body consumption information calculation mode of operation will be described. When entering this mode, first of all, at step S31, the control unit 38 controls to retrieve, from the storage unit 36, any one of the formulae (13) to (14) adaptable to body transition term corresponding to number of days from menstruation selected in the previous target information calculation mode. Then, at step S32, the body consumption information calculation unit 34 acts to substitute the data entered via the input unit 17 (current body weight, current body fat rate and age) and the previously calculated data for the formulae (11) and (12) retrieved from the storage unit 36 to calculate fat free mass and basal metabolism, and to substitute them for any one of the previously selected formulae (13) and (14) to calculate basal metabolism according to body transition term (luteal phase or follicular phase) corresponding to number of days from menstruation.

Then, at steps S33 to S37, the body consumption information calculation unit 34 similarly acts to substitute the previously calculated data and each type of data from the input unit 17, pedometer unit 32, the pulse rate measurement unit 20, the clock unit 37 and the storage unit 36 for the formulae (15) to (23) retrieved from the storage unit 36 to calculate the body consumption information (energy consumption due to exercise in follicular phase or luteal phase, total energy consumption in follicular phase or luteal phase, fat mass burned due to exercise in follicular phase or luteal phase, and total fat mass burned in follicular phase or luteal phase), taking into account of body transition term corresponding to number of days from menstruation, i.e. woman's physiological body transition term. More specifically, at step S33, any one of the formulae (16) and (17) is selected for calculating energy consumption due to exercise according to body transition term selected in the target information calculation mode. Then, at step S34, the formula (15) is used to calculate energy consumption due to exercise, and the previously selected formula (16) or (17) is used to calculate energy consumption due to exercise according to the body transition term. At step S35, the formula (18) or (19) is used to calculate total energy consumption according to body transition term, and at step S36, the formulae (20), (21) and (22) are used to calculate fat mass burned due to exercise according to body transition term, and at step S37, the formula (23) is used to calculate total fat mass burned according to body transition term.

The age · sex coefficient in the formula (15) is selected in table 1 as shown in Fig. 20 in such manner that "female" row is selected and one of the coefficients in that row is selected depending on age entered via the input unit 17. The sex coefficient in the formula (21) is defined as "0" which is the coefficient for female. The coefficients "a" to "r" have no concrete numerical value, here, but they are constant provided by regression analysis in creating each formula. The coefficients "f1", "f2", "g1", "g2", "h1", "h2", "i1", "i2" among those coefficients "a" to "r" has such magnitude relation that is represented by: "f1"<"f2"; "g1"< "g2"; "h1"< "h2"; and "i1"< "i2" (that is to say, the calculated result for body consumption information in luteal phase is greater than that for body consumption information in follicular phase).

Although one embodiment of the present invention has been described above, the present invention is not limited to such embodiment. For example, in the body consumption information calculation mode, as described above, the energy consumption due to exercise in follicular phase or luteal phase, total energy consumption in follicular phase or luteal phase, fat mass burned due to exercise in follicular phase or luteal phase, and total fat mass burned in follicular phase or luteal phase have been calculated as the body consumption information taking into account of woman's physiological body transition term. However, the present invention may be embodied by calculating one or more of such body consumption information.

Furthermore, the formula (12) has been used in the above-mentioned embodiment for calculating basal metabolism not taking into account of woman's physiological body transition term, but it may be possible that the following formulae (31) to (37) are stored in the storage unit 36 in advance, and any one of them is retrieved therefrom for calculation.

$$\text{Basal Metabolism [kcal/day]} = \text{Coefficient "A"} \times \text{Fat Free Mass [kg]} +$$
$$\text{Coefficient "B"} / \text{Age [years]} + \text{Coefficient "C"} \quad (31)$$

$$\text{Basal Metabolism [kcal/day]} = \text{Coefficient "D"} \times \text{Fat Free Mass [kg]} +$$
$$\text{Coefficient "E"} / \text{Age [years]} + \text{Coefficient "F"} \times \text{Weight [kg]} +$$
$$\text{Coefficient "G"} \quad (32)$$

$$\text{Basal Metabolism [kcal/day]} = \text{Coefficient "H"} \times \text{Fat Free Mass}^2 \text{ [kg}^2\text{]} +$$
$$\text{Coefficient "I"} \times \text{Fat Free Mass [kg]} +$$
$$\text{Coefficient "J"} / \text{Age [years]} + \text{Coefficient "K"} \quad (33)$$

$$\text{Basal Metabolism [kcal/day]} =$$
$$\text{Coefficient "L"} \times \text{Fat Free Mass [kg]} + \text{Coefficient "M"} \quad (34)$$

$$\text{Basal Metabolism [kcal/day]} = \text{Age} \cdot \text{Sex Coefficient} \times$$
$$(\text{Weight}^{0.444} \text{[kg]} \times \text{Height}^{0.663} \text{[cm]} \times 88.83) \quad (35)$$

$$\text{Basal Metabolism [kcal/day]} = \text{Basal Metabolism Reference for}$$
$$\text{Each of Sex} \cdot \text{Age Classes [kcal/kg/day]} \times \text{Weight [kg]} \quad (36)$$

$$\text{Basal Metabolism [kcal/day]} =$$
$$\text{Age Coefficient} \times \text{Weight [kg]} + \text{Coefficient "N"} \quad (37)$$

Furthermore, the formulae (13), (14), (16) to (19), (22) and (23) have been used in the above-mentioned embodiment for calculating body consumption information (energy consumption due to exercise, total energy consumption, fat mass burned due to exercise, and total fat mass burned) in follicular phase or luteal phase, but it may be possible that the following formulae (41) to (57) are stored in the storage unit 36 in advance for calculating body consumption information (energy consumption due to exercise, total energy consumption, fat mass burned due to exercise, and total fat mass burned) in menstruation term, diet term, before-and-after ovulation term, PMS (Premenstrual Syndrome) prevention term, or PMS term.

$$\text{Basal Metabolism in Menstruation Term [kcal/day]} =$$
$$\text{Coefficient "f3"} \times \text{Basal Metabolism [kcal/day]} + \text{Coefficient "g3"} \quad (41)$$

Basal Metabolism in Diet Term [kcal/day] =

Coefficient "f4" × Basal Metabolism [kcal/day] + Coefficient "g4"     (42)

Basal Metabolism in Before-and-After Ovulation Term [kcal/day] =

Coefficient "f5" × Basal Metabolism [kcal/day] + Coefficient "g5"     (43)

Basal Metabolism in PMS Prevention Term [kcal/day] =

Coefficient "f6" × Basal Metabolism [kcal/day] + Coefficient "g6"     (44)

Basal Metabolism in PMS Term [kcal/day] =

Coefficient "f7" × Basal Metabolism [kcal/day] + Coefficient "g7"     (45)

Energy Consumption due to Exercise in Menstruation Term [kcal/day] =

Coefficient "h3" × Energy Consumption due to

Exercise [kcal/day] + Coefficient "i3"     (46)

Energy Consumption due to Exercise in Diet Term [kcal/day] =

Coefficient "h4" × Energy Consumption due to

Exercise [kcal/day] + Coefficient "i4"     (47)

Energy Consumption due to Exercise in Before-and-After

Ovulation Term [kcal/day] =

Coefficient "h5" × Energy Consumption due to

Exercise [kcal/day] + Coefficient "i5"     (48)

Energy Consumption due to Exercise in PMS Prevention Term [kcal/day] =

Coefficient "h6" × Energy Consumption due to

Exercise [kcal/day] + Coefficient "i6"     (49)

Energy Consumption due to Exercise in PMS Term [kcal/day] =

Coefficient "h7" × Energy Consumption due to

Exercise [kcal/day] + Coefficient "i7"     (50)

Total Energy Consumption in Menstruation Term [kcal/day] =

Basal Metabolism in Menstruation Term [kcal/day] + Energy

Consumption due to Exercise in Menstruation Term [kcal/day]     (51)

Total Energy Consumption in Diet Term [kcal/day] =

Basal Metabolism in Diet Term [kcal/day] +

Energy Consumption due to Exercise in Diet Term [kcal/day]     (52)

Total Energy Consumption in Before-and-After Ovulation Term [kcal/day] =

Basal Metabolism in Before-and-After Ovulation Term [kcal/day] +

Energy Consumption due to Exercise in Before-and-After

Ovulation Term [kcal/day]     (53)

Total Energy Consumption in PMS Prevention Term [kcal/day] =

Basal Metabolism in PMS Prevention Term [kcal/day] +

Energy Consumption due to Exercise in PMS Prevention

Term [kcal/day]     (54)

Total Energy Consumption in PMS Term [kcal/day] =

Basal Metabolism in PMS Term [kcal/day] + Energy

Consumption due to Exercise in PMS Term [kcal/day]     (55)

Fat Mass Burned due to Exercise in Menstruation Term,

Diet Term, Before-and-After Ovulation Term,

PMS Prevention Term or PMS Term [g] =

Fat Burning Efficiency [%] × Energy Consumption due to Exercise in Menstruation Term, Diet Term, Before-and-After Ovulation Term, PMS Prevention Term or PMS Term [kcal/day] ╱

7.2 [kcal/g]     (56)

Total Fat Mass Burned in Menstruation Term,

Diet Term, Before-and-After Ovulation Term,

PMS Prevention Term or PMS Term [g] =

Fat Mass Burned due to Exercise [g] + Basal Metabolism

in Menstruation Term, Diet Term, Before-and-After Ovulation Term, PMS Prevention Term or PMS Term [kcal/day] ╱

7.2 [kcal/g]                                                    (57)

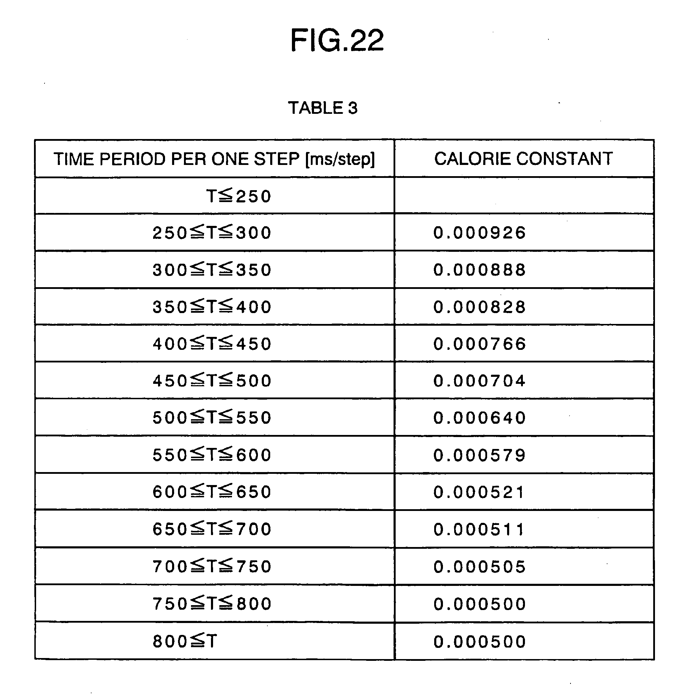

Moreover, in the above-mentioned embodiment, the formula (15) has been used for calculating energy consumption due to exercise, but it may possible that the following formulae (61) and (62) as well as table 3 in Fig. 22 illustrating relation between time period for one step and calorie constant are stored in the storage unit 36 in advance, and thereafter, the formula (61) is retrieved for calculation of time period for one step, on the basis of which the corresponding calorie constant is selected according to the table 3 in Fig, 22, and the formula (62) is retrieved for calculation of energy consumption due to exercise.

Time Period for One Step [ms/step] =

Walking Time [ms] ╱ Number of Steps [steps]                    (61)

Energy Consumption due to Exercise [kcal/day] =

Calorie Constant × Weight [kg] × Number of Steps [steps]       (62)

In addition, the formula (16) or (17) has been used for calculation of energy consumption due to exercise in follicular phase or luteal phase, but it may be possible that any one of the following formulae (63) to (65) are stored in the storage unit 36 in advance, and thereafter, it is retrieved for calculating energy consumption due to exercise in follicular phase or luteal phase. Furthermore, in those formulae (63) to (65), the basal metabolism in follicular phase or luteal phase may be replaced with the basal metabolism in menstruation term, diet term, before-and-after ovulation term, PMS prevention term, or PMS term to calculate the energy consumption due to exercise in menstruation term, diet term, before-and-after ovulation term, PMS prevention term, or PMS term.

Energy Consumption due to Exercise in Follicular or Luteal Phase [kcal/day] = (0.0140 × Fat Free Mass [kg] ╱

Height$^2$ [cm$^2$] − 0.0012 × Fat Rate [%] − 0.1254) × (Pulse Rate in Exercise

[pulses/min] − Pulse Rate at Rest [pulses/min]) + Basal Metabolism in

Follicular or Luteal Phase [kcal/day] ╱ 1440 + 0.1812          (63)

Energy Consumption due to Exercise in

$$\text{Follicular or Luteal Phase [kcal/day]} = 0.0051 \times (\text{Pulse Rate in Exercise}$$

$$[\text{pulses/min}] - \text{Pulse Rate at Rest [pulses/min]} + 15.5) \times \text{Basal}$$

$$\text{Metabolism in Follicular or Luteal Phase [kcal/day]} / 1440 \qquad (64)$$

Energy Consumption due to Exercise in

$$\text{Follicular or Luteal Phase [kcal/day]} = 0.075 \times (\text{Pulse Rate in}$$

$$\text{Exercise [pulses/min]} - \text{Pulse Rate at Rest [pulses/min]} + 15.5)$$

$$\times \text{Basal Metabolism in Follicular or Luteal Phase [kcal/day]} /$$

$$1440 + 0.0853 \qquad (65)$$

Moreover, fat burning efficiency calculated using the formula (21) has been substituted for to calculate fat mass burned due to exercise in follicular phase or luteal phase, but some other constant value (e.g. 30%, 40%, etc.) defined as the general fat burning efficiency in walking may be substituted for.

In addition, the formula (20) has been used for calculation of intensity of exercise, but it may be possible that the following formula (66) is stored in the storage unit 36 in advance and it is retrieved for calculation of the intensity of exercise.

$$\text{Intensity of Exercise [\%]} = \text{Pulse Rate in Exercise [pulses/min]} \times 100 /$$

$$(220 - \text{Age [years]} - \text{Pulse Rate at Rest [pulses/min]}) \qquad (66)$$

In the above mentioned embodiment the basic body data have been entered by entering the relevant numerical values via the input unit, but some measurement unit may be provided in the device for measuring the basic body data.

It is apparent from the foregoing that a device for providing body consumption information of a woman according to the present invention is configured to calculate body consumption information by taking into account of woman's body transition term, which makes it possible to get such precise body consumption information for a woman that is effective for control of consumption in the body or for health care of the woman.

Furthermore, the device is configured to provide target information by taking into account of woman's body transition term and to provide some advice information in comparison to that target information, which is effective for continuation of more proper health care of the woman.

**Claims**

1. A device for providing body consumption information of a woman, comprising:

   a basic body data acquisition unit,
   a number-of-days-after-menstruation acquisition unit;
   a body transition term data storage unit;
   a body consumption information calculation formula storage unit;
   a body transition term selection unit;
   a body consumption information calculation unit; and
   a body consumption information display unit, wherein
   said basic body data acquisition unit acquires basic body data relating to consumption in the body,
   said number-of-days-after-menstruation acquisition unit acquires the number of days that have elapsed since a menstruation start day,

said body transition term data storage unit stores, in advance, the body transition term data that is formed by correlation of woman's physiological body transition terms with the number of days after menstruation,
said body consumption information calculation formula storage unit stores, in advance, an calculation formula for getting information about consumption in the body, taking into account of woman's physiological body transition term, for each of the body transition terms,
said body transition term selection unit selects a woman's physiological body transition term corresponding to the number of days after menstruation acquired by said number-of days-after-menstruation acquisition unit on the basis of the body transition term data stored in said body transition term data storage unit,
said body consumption information calculation unit calculates information about consumption in the body, taking into account of woman's physiological body transition terms, by substituting the basic body data relating to body information acquired by said basic body data acquisition unit for the calculation formula retrieved from said body consumption information calculation formula storage unit according to the woman's physiological body transition term selected by said body transition term selection unit, and
said body consumption information display unit displays the body information, taking into account of woman's physiological body transition term, calculated by said body consumption information calculation unit.

2.  A device for providing body consumption information of a woman according to claim 1 in which it further comprises:

    a target basic data acquisition unit;
    a target information calculation formula storage unit;
    a target information calculation unit; and
    a target information display unit, wherein
    said target basic data acquisition unit acquires a target basic data relating to consumption in the body,
    said target information calculation formula storage unit stores, in advance, an calculation formula for providing target information, taking into account of woman's physiological body transition term, for each of body transition terms,
    said target information calculation unit calculates target information, taking into account of woman's physiological body transition term, by substituting the target basic data relating to consumption in the body acquired by said target basic data acquisition unit for the calculation formula retrieved from said target information calculation formula storage unit according to the woman's physiological body transition term selected by said body transition term selection unit, and
    said target information display unit displays the target information, taking into account of woman's physiological body transition term, calculated by said target information calculation unit.

3.  A device for providing body consumption information of a woman according to claim 2 in which it further comprises an advice information display unit, said advice information display unit displays advice information for consumption in the body, together with the body consumption information calculated by said body consumption information calculation unit in comparison with the target information calculated by said target information calculation unit.

4.  A device for providing body consumption information of a woman according to any one of claims 1 to 3 in which it further comprises an intensity-of-exercise measurement unit for measuring how much an exercise has been done, said body consumption information calculation unit calculates the body consumption information, taking into account of the intensity of exercise measured by said intensity-of-exercise measurement unit.

5.  A device for providing body consumption information of a woman according to claim 4 in which it further comprises a time-of-exercise measurement unit for measuring how long an exercise has been done, said body consumption information calculation unit calculates the body consumption information, taking into account of the time duration of exercise measured by said time-of-exercise measurement unit.

6.  A device for providing body consumption information of a woman according to claim 4 or 5 in which said intensity of exercise is represented by the number of steps.

7.  A device for providing body consumption information of a woman according to claim 6 in which said time duration of exercise is represented by the walling time.

8.  A device for providing body consumption information of a woman according to claim 6 in which it further comprises:

    a pulse rate measurement unit; and

a pitch guide tone generator unit, wherein
said pulse rate measurement unit measures a pulse rate of a woman under test, and
said pitch guide tone generator unit generates pitch guide tone for prompting the woman under test to walk at pulse rate of fixed and safety range according to the pulse rate measured by said pulse rate measurement unit.

9. A device for providing body consumption information of a woman according to any one of claims 1 to 8 in which said number-of-days-after-menstruation acquisition unit comprises an input section and a clock section, said input unit enters the previous menstruation date and the current date, and said clock section calculates the number of days after menstruation, which means the number of days elapsed from the previous menstruation date to the current date, depending on the previous menstruation date and the current date entered by said input unit.

10. A device for providing body consumption information of a woman according to any one of claims 1 to 9 in which said body transition term includes follicular phase and luteal phase.

11. A device for providing body consumption information of a woman according to any one of claims 1 to 9 in which said body transition term includes menstruation term, diet term, before-and-after ovulation term, PMS prevention term, and PMS term.

12. A device for providing body consumption information of a woman according to any one of claims 1 to 11 in which said body consumption information includes at least one of the followings: basal metabolism, energy consumption due to exercise, total energy consumption, fat mass burned due to exercise, and total burned fat mass.

13. A device for providing body consumption information of a woman according to any one of claims 6 to 12 in which said target information includes target number of steps.

**Patentansprüche**

1. Vorrichtung zum Bereitstellen von Körper-Energieverbrauchsinformationen einer Frau, die umfasst:

eine Einheit zum Ermitteln von Basis-Körperdaten,
eine Einheit zum Ermitteln der Anzahl von Tagen nach der Menstruation;
eine Einheit zum Speichern von Körper-Übergangszeitraumdaten;
eine Einheit zum Speichern einer Formel zum Berechnen von Körper-Energieverbrauchsinformationen;
eine Einheit zum Auswählen eines Körper-Übergangszeitraums;
eine Einheit zum Berechnen von Körper-Energieverbrauchsinformationen; und
eine Einheit zum Anzeigen von Körper-Energieverbrauchsinformationen, wobei
die Einheit zum Ermitteln von Basis-Körperdaten Basis-Körperdaten ermittelt, die sich auf Energieverbrauch im Körper beziehen,
die Einheit zum Erfassen der Anzahl von Tagen nach der Menstruation die Anzahl von Tagen erfasst, die seit einem Anfangstag der Menstruation vergangen sind,
die Einheit zum Speichern der Körper-Übergangszeitraumdaten im Voraus die Körper-Übergangszeitraumdaten speichert, die erzeugt werden, indem physiologische Körper-Übergangszeiträume einer Frau zu der Anzahl von Tagen nach der Menstruation korreliert werden,
die Einheit zum Speichern einer Formel zum Berechnen von Körper-Energieverbrauchsinformationen im Voraus eine Berechnungsformel zum Ermitteln von Informationen über Energieverbrauch in dem Körper, die einen physiologischen Körper-Übergangszeitraum einer Frau berücksichtigen, für jeden der Körper-Übergangszeiträume speichert,
die Einheit zum Auswählen eines Körper-Übergangszeitraums einen physiologischen Körper-Übergangszeitraum einer Frau, der der Anzahl von Tagen nach der Menstruation entspricht, die durch die Einheit zum Ermitteln der Anzahl von Tagen nach der Menstruation ermittelt wird, auf Basis der in der Einheit zum Speichern von Körper-Übergangszeitraumdaten gespeicherten Körper-Übergangszeitraumdaten auswählt,
die Einheit zum Berechnen von Körper-Energieverbrauchsinformationen Informationen über Energieverbrauch in dem Körper, die physiologische Körper-Übergangszeiträume berücksichtigen, berechnet, indem sie die Basis-Körperdaten, die sich auf die durch die Einheit zum Erfassen von Körper-Basisdaten ermittelten Körperinformationen beziehen, in die von der Einheit zum Speichern einer Formel zum Berechnen von Körper-Energieverbrauchsinformationen entsprechend dem durch die Einheit zum Auswählen des Körper-Übergangszeitraums ausgewählten physiologischen Körper-Übergangszeitraum abgerufene Berechnungsformel einsetzt, und

die Einheit zum Anzeigen von Körper-Verbrauchsinformationen die durch die Einheit zum Berechnen von Körper-Energieverbrauchsinformationen berechneten Körperinformationen anzeigt, die einen physiologischen Körper-Übergangszeitraum berücksichtigen.

2. Vorrichtung zum Bereitstellen von Körper-Energieverbrauchsinformationen einer Frau nach Anspruch 1, wobei sie des Weiteren umfasst:

eine Einheit zum Erfassen von Ziel-Basisdaten;
eine Einheit zum Speichern einer Formel zum Berechnen von Zielinformationen;
eine Einheit zum Berechnen von Zielinformationen; und
eine Einheit zum Anzeigen von Zielinformationen, wobei
die Einheit zum Erfassen von Ziel-Basisdaten Ziel-Basisdaten bezüglich des Energieverbrauchs im Körper erfasst,
die Einheit zum Speichern einer Formel zum Berechnen von Zielinformationen im Voraus eine Berechnungsformel zum Bereitstellen von Zielinformationen, die einen physiologischen Körper-Übergangszeitraum einer Frau berücksichtigen, für jeden Körper-Übergangszeitraum speichert,
die Einheit zum Berechnen von Zielinformationen Zielinformationen, die einen physiologischen Körper-Übergangszeitraum einer Frau berücksichtigen, berechnet, indem sie die durch die Einheit zum Erfassen von Ziel-Basisdaten erfassten Ziel-Basisdaten bezüglich des Energieverbrauchs in dem Körper entsprechend dem durch die Einheit zum Auswählen des Körper-Übergangszeitraums ausgewählten physiologischen Körper-Übergangszeitraums der Frau in die von der Einheit zum Speichern der Formel zum Berechnen der Zielinformationen abgerufene Berechnungsformel einsetzt, und
die Einheit zum Anzeigen der Zielinformationen die durch die Einheit zum Berechnen der Zielinformationen berechneten Zielinformationen anzeigt, die einen physiologischen Körper-Übergangszeitraum der Frau berücksichtigen.

3. Vorrichtung zum Bereitstellen von Körper-Energieverbrauchsinformationen einer Frau nach Anspruch 2, wobei sie des Weiteren eine Einheit zum Anzeigen von Empfehlungsinformationen umfasst und die Einheit zum Anzeigen von Empfehlungsinformationen Empfehlungsinformationen zum Energieverbrauch in dem Körper zusammen mit den durch die Einheit zum Berechnen von Körper-Energieverbrauchsinformationen berechneten Körper-Energieverbrauchsinformationen im Vergleich zu den durch die Einheit zum Berechnen von Zielinformationen berechneten Zielinformationen anzeigt.

4. Vorrichtung zum Bereitstellen von Körper-Energieverbrauchsinformationen einer Frau nach einem der Ansprüche 1 bis 3, wobei sie des Weiteren eine Einheit zum Messen von Trainingsintensität umfasst, mit der gemessen wird, wie viel Training absolviert worden ist, und die Einheit zum Berechnen von Körper-Energieverbrauchsinformationen die Körper-Energieverbrauchsinformationen unter Berücksichtigung der durch die Einheit zum Messen von Trainingsintensität gemessenen Trainingsintensität berechnet.

5. Vorrichtung zum Bereitstellen von Körper-Energieverbrauchsinformationen einer Frau nach Anspruch 4, wobei sie des Weiteren eine Einheit zum Messen einer Trainingszeit umfasst, mit der gemessen wird, wie lange trainiert worden ist, und die Einheit zum Berechnen von Körper-Energieverbrauchsinformationen die Körper-Energieverbrauchsinformationen unter Berücksichtigung der durch die Einheit zum Messen von Trainingszeit gemessene Trainingszeitdauer berechnet.

6. Vorrichtung zum Bereitstellen von Körper-Energieverbrauchsinformationen einer Frau nach Anspruch 4 oder 5, wobei die Trainingsintensität durch die Anzahl von Schritten dargestellt wird.

7. Vorrichtung zum Bereitstellen von Körper-Energieverbrauchsinformationen einer Frau nach Anspruch 6, wobei die Trainingszeitdauer durch die Gehzeit dargestellt wird.

8. Vorrichtung zum Bereitstellen von Körper-Energieverbrauchsinformationen einer Frau nach Anspruch 6, wobei sie des Weiteren umfasst:

eine Einheit zum Messen der Pulsfrequenz; und
eine Einheit zum Erzeugen eines Intervall-Führungstons (pitch guide tone), wobei
die Einheit zum Messen der Pulsfrequenz eine Pulsfrequenz einer Frau im Test misst, und
die Einheit zum Erzeugen eines Intervall-Leittons einen Intervall-Leitton erzeugt, der die Frau im Test entsprechend der durch die Einheit zum Messen der Pulsfrequenz gemessenen Pulsfrequenz veranlasst, bei einer Impulsfrequenz eines festen und sicheren Bereiches zu gehen.

**9.** Vorrichtung zum Bereitstellen von Körper-Energieverbrauchsinformationen einer Frau nach einem der Ansprüche 1 bis 8, wobei die Einheit zum Erfassen der Anzahl von Tagen nach der Menstruation einen Eingabeabschnitt und einen Uhrabschnitt umfasst, der Eingabeabschnitt das Datum der vorhergehenden Menstruation und das aktuelle Datum eingibt, der Uhrabschnitt die Anzahl von Tagen nach der Menstruation, d.h. die Anzahl von Tagen, die seit dem Datum der vorhergehenden Menstruation bis zum aktuellen Datum vergangen sind, in Abhängigkeit von dem Datum der vorhergehenden Menstruation und dem aktuellen Datum, die durch die Eingabeeinheit eingegeben wurden, berechnet.

**10.** Vorrichtung zum Bereitstellen von Körper-Energieverbrauchsinformationen einer Frau nach einem der Ansprüche 1 bis 9, wobei der Körper-Übergangszeitraum eine Follikularphase und eine Lutealphase einschließt.

**11.** Vorrichtung zum Bereitstellen von Körper-Energieverbrauchsinformationen einer Frau nach einem der Ansprüche 1 bis 9, wobei der Körper-Übergangszeitraum einen Menstruationszeitraum, Aufbauzeitraum (diet term), Zeitraum vor und nach dem Eisprung, Zeitraum zum Verhindern des prämenstruellen Syndroms und Zeitraum des prämenstruellen Syndroms einschließt.

**12.** Vorrichtung zum Bereitstellen von Körper-Energieverbrauchsinformationen einer Frau nach einem der Ansprüche 1 bis 11, wobei die Körper-Energieverbrauchsinformationen wenigstens eine der folgenden Informationen einschließen: Basalstoffwechsel, Energieverbrauch aufgrund von Training, Gesamtenergieverbrauch, aufgrund von Training verbrannte Fettmasse und insgesamt verbrannte Fettmasse.

**13.** Vorrichtung zum Bereitstellen von Körper-Energieverbrauchsinformationen einer Frau nach einem der Ansprüche 6 bis 12, wobei die Zielinformationen eine Ziel-Anzahl von Schritten einschließen.

## Revendications

**1.** Dispositif pour délivrer des informations de consommation du corps d'une femme, comportant :

une unité d'acquisition de données basiques du corps;
une unité d'acquisition de nombre de jours après les règles ;
une unité de stockage de données de terme de transition de corps ;
une unité de stockage de formule de calcul d'information de consommation de corps ;
une unité de sélection de terme de transition de corps ;
une unité de calcul d'information de consommation de corps ; et
une unité d'affichage d'informations de consommation de corps, dans lequel
ladite unité d'acquisition de données basiques du corps acquiert des données basiques du corps ayant trait à la consommation dans le corps,
ladite unité d'acquisition de nombre de jours après les règles acquiert le nombre de jours qui se sont écoulés depuis le jour du début des règles,
ladite unité de stockage de données de terme de transition de corps mémorise, à l'avance, les données de terme de transition du corps qui sont formées par corrélation des termes de transition du corps physiologique de la femme avec le nombre de jours après les règles,
ladite unité de stockage de formule de calcul d'information de consommation de corps mémorise, à l'avance, une formule de calcul pour obtenir une information concernant la consommation dans le corps, prenant en compte le terme de transition de corps physiologique de la femme, pour chacun des termes de transition du corps,
ladite unité de sélection de terme de transition de corps sélectionne un terme de transition de corps physiologique de la femme correspondant au nombre de jours après les règles acquis par ladite unité d'acquisition de nombre de jours après les règles en fonction des données de terme de transition de corps mémorisées dans ladite unité de stockage de données de terme de transition de corps,
ladite unité de calcul d'information de consommation de corps calcule une information concernant la consommation dans le corps, prenant en compte les termes de transition de corps physiologique de la femme, en substituant les données basiques de corps ayant trait à l'information de corps acquise par ladite unité d'acquisition de données basiques de corps pour la formule de calcul récupérée depuis l'unité de stockage de formule de calcul d'information de consommation de corps selon le terme de transition de corps physiologique de la femme sélectionné par ladite unité de sélection de terme de transition de corps, et

ladite unité d'affichage d'informations de consommation de corps affiche l'information de corps, en prenant en compte le terme de transition de corps physiologique de la femme, calculé par ladite unité de calcul d'information de consommation de corps.

**2.** Dispositif pour délivrer des informations de consommation de corps d'une femme selon la revendication 1, comportant en outre :

une unité d'acquisition de données basiques cibles;

une unité de stockage de formule de calcul d'informations cibles ;

une unité de calcul d'informations cibles ; et

une unité d'affichage d'informations cibles, dans lequel

ladite unité d'acquisition de données basiques cibles acquiert des données basiques cibles ayant trait à la consommation dans le corps,

ladite unité de stockage de formule de calcul d'informations cibles mémorise, à l'avance, une formule de calcul pour délivrer une information cible, prenant en compte un terme de transition de corps physiologique de la femme, pour chacun des termes de transition de corps,

ladite unité de calcul d'informations cibles calcule une information cible, prenant en compte un terme de transition de corps physiologique de la femme, en substituant les données basiques cibles ayant trait à la consommation dans le corps acquises par ladite unité d'acquisition de données basiques cibles pour la formule de calcul récupérée depuis l'unité de stockage de formule de calcul d'informations cibles conformément au terme de transition de corps physiologique de la femme sélectionné par ladite unité de sélection de terme de transition de corps, et

ladite unité d'affichage d'informations cibles affiche l'information cible, prenant en compte un terme de transition de corps physiologique de la femme, calculé par ladite unité de calcul d'informations cibles.

**3.** Dispositif pour délivrer des informations de consommation de corps d'une femme selon la revendication 2, comportant en outre une unité d'affichage d'informations de conseil, ladite unité d'affichage d'informations de conseil affiche l'information de conseil pour la consommation dans le corps, conjointement avec l'information de consommation du corps calculée par ladite unité de calcul d'information de consommation de corps en comparaison avec l'information cible calculée par ladite unité de calcul d'informations cibles.

**4.** Dispositif pour délivrer des informations de consommation de corps d'une femme selon l'une quelconque des revendications 1 à 3, comportant en outre une unité de mesure d'intensité d'exercice pour mesurer la quantité d'un exercice qui a été effectué, ladite unité de calcul d'information de consommation de corps calcule l'information de consommation de corps, en prenant en compte l'intensité de l'exercice mesurée par ladite unité de mesure d'intensité d'exercice.

**5.** Dispositif pour délivrer des informations de consommation de corps d'une femme selon la revendication 4, comportant en outre une unité de mesure de temps d'exercice pour mesurer le temps pendant lequel un exercice a été effectué, ladite unité de calcul d'information de consommation de corps calcule l'information de consommation de corps, en prenant en compte la durée de l'exercice mesuré par ladite unité de mesure de temps d'exercice.

**6.** Dispositif pour délivrer une information de consommation de corps d'une femme selon la revendication 4 ou 5, dans lequel ladite intensité d'exercice est représentée par le nombre de pas.

**7.** Dispositif pour délivrer une information de consommation de corps d'une femme selon la revendication 6, dans lequel ladite durée de l'exercice est représentée par le temps de marche.

**8.** Dispositif pour délivrer des informations de consommation de corps d'une femme selon la revendication 6, comportant en outre :

une unité de mesure de fréquence de pulsation ; et

une unité de générateur de son de guidage de pas, dans lequel

ladite unité de mesure de fréquence de pulsation mesure la fréquence de pulsation d'une femme faisant le test, et

ladite unité de générateur de son de guidage de pas génère un son de guidage de pas pour inciter la femme faisant le test à marcher à la fréquence de pulsation d'une plage déterminée et sûre selon la fréquence de pulsation mesurée par l'unité de mesure de fréquence de pulsation.

**9.** Dispositif pour délivrer des informations de consommation de corps d'une femme selon l'une quelconque des revendications 1 à 8, dans lequel ladite unité d'acquisition de nombre de jours après les règles comprend une section d'entrée et une section d'horloge, ladite unité d'entrée entre la date précédente des règles et la date courante, et ladite section d'horloge calcule le nombre de jours après les règles, qui signifie le nombre de jours écoulés depuis la date précédente des règles jusqu'à la date courante, en fonction de la date précédente des règles et de la date courante entrée par ladite unité d'entrée.

**10.** Dispositif pour délivrer une information de consommation de corps d'une femme selon l'une quelconque des revendications 1 à 9, dans lequel ledit terme de transition de corps comprend la phase folliculaire et la phase lutéale.

**11.** Dispositif pour délivrer une information de consommation de corps d'une femme selon l'une quelconque des revendications 1 à 9, dans lequel ledit terme de transition de corps comprend le terme des règles, le terme de régime, le terme avant et après ovulation, le terme de prévention de PMS, et le terme de PMS.

**12.** Dispositif pour délivrer une information de consommation de corps d'une femme selon l'une quelconque des revendications 1 à 11, dans lequel ladite information de consommation de corps comprend au moins l'une des informations suivantes : le métabolisme basal, la consommation d'énergie due à l'exercice, la consommation d'énergie totale, la masse de graisses brûlées grâce à l'exercice, et la masse de graisses brûlées totale.

**13.** Dispositif pour délivrer une information de consommation de corps d'une femme selon l'une quelconque des revendications 6 à 12, dans lequel ladite information cible comprend le nombre cible de pas.

# FIG.1

# FIG.2

20,30

1

10

2

# FIG.3

# FIG.4

START
(TURN ON START BUTTON)

S1 — IS SETTING BUTTON DEPRESSED?

YES → BODY INFORMATION AND OTHER BASIC DATA SETTING AND REGISTRATION MODE

NO

TARGET INFORMATION CALCULATION MODE

DISPLAY TARGET INFORMATION

S2 — IS "YES" IS SELECTED?

NO

YES

GENERATE PITCH GUIDE TONE AND MEASURE NUMBER OF STEPS AND WALKING TIME — S3

BODY CONSUMPTION INFORMATION CALCULATION MODE

DISPLAY BODY CONSUMPTION INFORMATION

S4 — IS SWITCHING BUTTON DEPRESSED?

NO

YES

S5 — SWITCH DISPLAY INFORMATION

# FIG.5

BODY INFORMATION AND OTHER
BASIC DATA SETTING AND
REGISTRATION MODE

S11 — DISPLAY INPUT
SCREEN FOR ENTERING
BASIC BODY DATA

S12 — STORE ENTERED BASIC BODY DATA

S13 — DISPLAY INPUT SCREEN
FOR ENTERING BASIC
MENSTRUATION DATA

S14 — STORE ENTERED BASIC MENSTRUATION
DATA

S15 — DISPLAY INPUT SCREEN
FOR ENTERING TARGET
BASIC DATA

S16 — STORE ENTERED TARGET BASIC DATA

S17 — DISPLAY SCREEN FOR
MEASURING PULSE RATE
AT REST CONDITION

S18 — MEASURE AND STORE PULSE RATE AT
REST CONDITION

RETURN

# FIG.6

```
    ┌─────────────────────────┐
    │  TARGET INFORMATION     │
    │  CALCULATION MODE       │
    └─────────────────────────┘
                │
                ▼
S21  ┌─────────────────────────────────────────┐
     │      CALCULATE TARGET FAT MASS          │
     └─────────────────────────────────────────┘
                │
                ▼
S22  ┌─────────────────────────────────────────┐
     │  CALCULATE TARGET FAT MASS REDUCTION    │
     └─────────────────────────────────────────┘
                │
                ▼
S23  ┌─────────────────────────────────────────┐
     │   CALCULATE TARGET FAT BURNED ENERGY    │
     └─────────────────────────────────────────┘
                │
                ▼
S24  ┌─────────────────────────────────────────┐
     │  CALCULATE NUMBER OF DAYS IN TARGET TERM│
     └─────────────────────────────────────────┘
                │
                ▼
S25  ┌─────────────────────────────────────────┐
     │     CALCULATE TARGET FAT BURNED         │
     │          ENERGY PER DAY                 │
     └─────────────────────────────────────────┘
                │
                ▼
S26  ┌─────────────────────────────────────────┐
     │  CALCULATE ENERGY CONSUMPTION PER STEP  │
     └─────────────────────────────────────────┘
                │
                ▼
S27  ┌─────────────────────────────────────────┐
     │ CALCULATE TARGET NUMBER OF STEPS PER DAY│
     └─────────────────────────────────────────┘
                │
                ▼
S28  ┌─────────────────────────────────────────┐
     │ CALCULATE AND SELECT BODY TRANSITION    │
     │ TERM FROM MENSTRUATION AND CALCULATE    │
     │ AVERAGE NUMBER OF DAYS IN MENSTRUATION  │
     │ PERIOD                                  │
     └─────────────────────────────────────────┘
                │
                ▼
S29  ┌─────────────────────────────────────────┐
     │ CALCULATE TARGET NUMBER OF STEPS PER DAY│
     │ ACCORDING TO BODY TRANSITION TERM FROM  │
     │ MENSTRUATION                            │
     └─────────────────────────────────────────┘
                │
                ▼
         ┌──────────────┐
         │    RETURN    │
         └──────────────┘
```

# FIG.7

```
          ┌──────────────────────┐
          │   BODY CONSUMPTION    │
          │     INFORMATION       │
          │   CALCULATION MODE    │
          └──────────────────────┘
                     │
                     ▼
```

S31 — SELECT FORMULA FOR BASAL METABOLISM ACCORDING TO BODY TRANSITION TERM FROM MENSTRUATION

S32 — CALCULATE BASAL METABOLISM ACCORDING TO BODY TRANSITION TERM FROM MENSTRUATION

S33 — SELECT FORMULA FOR ENERGY CONSUMPTION DUE TO EXERCISE ACCORDING TO BODY TRANSITION TERM FROM MENSTRUATION

S34 — CALCULATE ENERGY CONSUMPTION DUE TO EXERCISE ACCORDING TO BODY TRANSITION TERM FROM MENSTRUATION

S35 — CALCULATE TOTAL ENERGY CONSUMPTION ACCORDING TO BODY TRANSITION TERM FROM MENSTRUATION

S36 — CALCULATE FAT MASS BURNED DUE TO EXERCISE ACCORDING TO BODY TRANSITION TERM FROM MENSTRUATION

S37 — CALCULATE TOTAL FAT MASS BURNED ACCORDING TO BODY TRANSITION TERM FROM MENSTRUATION

```
          ┌──────────────────────┐
          │        RETURN         │
          └──────────────────────┘
```

29

# FIG.8

INPUT YOUR BASIC BODY DATA.

HEIGHT     :   X X X cm

WEIGHT     :   X☒X kg

AGE          :    X X YEARS OLD

FAT RATE   :    X X %

                                ENTER

# FIG.9

INPUT YOUR BASIC MENSTRUATION DATA.

CURRENT DATE              : MONTH: XX DAY: XX YEAR: XXXX

PREVIOUS
MENSTRUATION DATE     : MONTH: XX DAY: XX YEAR: XXXX

NUMBER   OF
DAYS FOR PREVIOUS
MENSTRUATION PERIOD :                   ☒X DAYS

NUMBER OF DAYS IN
PREVIOUS
MENSTRUATION TERM    :                    XX DAYS

                                ENTER

# FIG.10

INPUT YOUR TARGET BASIC DATA.

TARGET FAT RATE : ⬚X %

ANTICIPATED DATE
TO ATTAIN TARGET : MONTH: XX DAY: XX YEAR: XXXX

ENTER

# FIG.11

MEASURE YOUR PULSE RATE AT REST CONDITION.

| MEASURE |
|---------|

PULSE RATE AT REST : XXX PULSES/MIN.

ENTER

# FIG.12

M    25TH DAY / 28 DAYS

IN LUTEAL AND PMS PHASE

# FIG.13

TARGET NUMBER OF
STEPS, TODAY          START TO WALK?

8000STEPS          YES

FOR SKIN ROUGHEN, TAKE VITAMIN B & C.
FOR NERVOUS CONDITION,
TAKE CALCIUM & MAGNESIUM.

IN TENDENCY OF GETTING
NERVOUS A DEEP BREATH IS EFFECTIVE⋯☆

RECOMMENDED FOOD:
FERMENTED SOYBEAN,
SPINACH AND BANANA

## FIG.14

AAAA STEPS

YYYY kcal

BBB g

## FIG.15

TARGET NUMBER OF STEPS, TODAY: TTT STEPS

NUMBER OF STEPS WALKED UP TO NOW, TODAY: AAAA STEPS

ADVICE:
YOU EXCEEDED TARGET NUMBER OF STEPS, TODAY.
KEEP SUCH PACE FOR REMAINING 25 DAYS UP TO THE ANTICIPATED DATE TO ATTAIN TARGET FAT RATE.

## FIG.16

GRAPH FOR SHOWING FAT MASS
BURNED DUE TO EXERCISE

M     25TH DAY / 28 DAYS

## FIG.17

| M | CYCLE |
|---|---|
| JULY. 12. 2001 | 28DAYS |
| AUG. 11. 2001 | 29DAYS |
| SEP. 9. 2001 | 29DAYS |

# FIG.18

ANTICIPATED DATE FOR START
OF FUTURE MENSTRUATION

OCT. 2~OCT. 10

OCT. 30~NOV. 5

NOV. 29~DEC. 5

# FIG.19

| | |
|---|---|
| BASAL METABOLISM | ZZZZ kcal |
| ENERGY CONSUMPTION DUE TO EXERCISE | YYYY kcal |
| TOTAL ENERGY CONSUMPTION | XXXX kcal |
| FAT MASS BURNED DUE TO EXERCISE | WWW g |
| TOTAL FAT MASS BURNED | UUU g |

# FIG.20

TABLE 1

| | LESS THAN 20 YEARS OLD | 20 TO 39 YEARS OLD | 40 TO 49 YEARS OLD | 50 TO 59 YEARS OLD |
|---|---|---|---|---|
| FEMALE | 0.083 | 0.080 | 0.072 | 0.070 |
| MALE | 0.092 | 0.087 | 0.079 | 0.077 |

# FIG.21

TABLE 2

| NUMBER OF DAYS FROM MENSTRUATION | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|---|---|---|---|---|---|---|---|---|---|---|---|---|
| BODY TRANSITION TERMS | MENSTRUATION TERM | | | | DIET TERM | | | | | | | | BEFORE-AND-AFTER OVULATION TERM | | | | PMS PREVENTION TERM | | | | PMS TERM | | | | | | |
| | FOLLICULAR PHASE | | | | | | | | | | | | | LUTEAL PHASE | | | | | | | | | | | | | | |

# FIG.22

TABLE 3

| TIME PERIOD PER ONE STEP [ms/step] | CALORIE CONSTANT |
|---|---|
| T≦250 | |
| 250≦T≦300 | 0.000926 |
| 300≦T≦350 | 0.000888 |
| 350≦T≦400 | 0.000828 |
| 400≦T≦450 | 0.000766 |
| 450≦T≦500 | 0.000704 |
| 500≦T≦550 | 0.000640 |
| 550≦T≦600 | 0.000579 |
| 600≦T≦650 | 0.000521 |
| 650≦T≦700 | 0.000511 |
| 700≦T≦750 | 0.000505 |
| 750≦T≦800 | 0.000500 |
| 800≦T | 0.000500 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 8052119 A

- JP 2000316833 A